# FASCICULE DE BREVET EUROPEEN

(11) **EP 1 377 660 B1**
(45) Date de publication et mention de la délivrance du brevet: **13.01.2016**
(21) Numéro de dépôt: 02759818.4
(22) Date de dépôt: 05.04.2002
(51) Int. Cl.: C12N 1/00

(54) **VACCIN CONTRE LE VIRUS DE LA FIEVRE DU NIL**
IMPFSTOFF GEGEN WEST NILE VIRUS
VACCINE AGAINST THE NILE FEVER VIRUS

(30) Priorité: 06.04.2001 FR 0104737
(43) Date de publication de la demande: 07.01.2004
(60) Demande divisionnaire: 15162832.8
(73) Titulaire: MERIAL, 69007 Lyon (FR)
(72) Inventeur: LOOSMORE, Sheena, May, Aurora, Ontario L4G 4R4 (CA); AUDONNET, Jean-Christophe, Francis, F-69006 Lyon (FR); MINKE, Jules Maarten, 69960 Corbas (FR)
(74) Mandataire: Cabinet Plasseraud
(86) Numéro de dépôt international: PCT/FR2002/001200
(87) Numéro de publication internationale: WO 2002/081621

(56) Documents cités:
- WO-A-00/12128
- WENGLER G ET AL: "AN ANALYSIS OF THE ANTIBODY RESPONSE AGAINST WEST NILE VIRUS E PROTEIN PURIFIED BY SDS-PAGE INDICATES THAT THIS PROTEIN DOES NOT CONTAIN SEQUENTIAL EPITOPES FOR EFFICIENT INDUCTION OF NEUTRALIZING ANTIBODIES" JOURNAL OF GENERAL VIROLOGY, vol. 70, no. 4, 1989, pages 987-992, XP001050460 ISSN: 0022-1317
- OCAZIONEZ JIMENEZ R ET AL: "Recombinant plasmid expressing a truncated dengue-2 virus E protein without co-expression of prM protein induces partial protection in mice" VACCINE, BUTTERWORTH SCIENTIFIC. GUILDFORD, GB, vol. 19, no. 6, 8 novembre 2000 (2000-11-08), pages 648-654, XP004219903 ISSN: 0264-410X
- LUSTIG S ET AL: "A LIVE ATTENUATED WEST NILE VIRUS STRAIN AS A POTENTIAL VETERINARY VACCINE", VIRAL IMMUNOLOGY, MARY ANN LIEBERT, INC., NEW YORK, US, vol. 13, no. 4, 1 December 2000 (2000-12-01), pages 401-410, XP008018324, ISSN: 0882-8245
- NAM J H ET AL: "Protection against lethal Japanese encephalitis virus infection of mice by immunization with the highly attenuated MVA strain of vaccinia virus expressing JEV prM and E genes", VACCINE, ELSEVIER LTD, GB, vol. 17, no. 3, 1 February 1999 (1999-02-01), pages 261-268, XP027303816, ISSN: 0264-410X [retrieved on 1999-02-01]

### SEQUENCE LISTING

<110> Merial
<120> Vaccin contre le virus de la fièvre du Nil.
<130> XXXX
<160> 29
<170> Patent In version 3.0
<210> 1
   <211> 30
   <212> DNA
   <213> Artificial
<220>
   <223> oligonucleotide
<400> 1
   ttttttgaat tcgttaccct ctctaacttc
   30
<210> 2
   <211> 33
   <212> DNA
   <213> Artificial
<220>
   <223> oligonucléotides
<400> 2
   tttttttcta gattacctcc gactgcgtct tga
   33
<210> 3
   <211> 40
   <212> DNA
   <213> Artificial
<220>
   <223> oligonucleotide
<400> 3
   gatctgcagc acgtgtctag aggatatcga attcgcggcc 40
<210> 4
   <211> 40
   <212> DNA
   <213> Artificial
<220>
   <223> oligonucleotide
<400> 4
   gatccgcggc cgcgaattcg atatcctcta gacacgtgct 40
<210> 5
   <211> 30
   <212> DNA
   <213> Artificial
<220>
   <223> oligonucleotide
<400> 5
   ttttttgaat tctcactgac agtgcagaca 30
<210> 6
   <211> 33
   <212> DNA
   <213> Artificial
<220>
   <223> oligonucleotide
<400> 6
   tttttttcta gattagctgt aagctggggc cac 33
<210> 7
   <211> 30
   <212> DNA
   <213> Artificial
<220>
   <223> oligonucleotide
<400> 7
   ttttttgaat tcttcaactg ccttggaatg
   30
<210> 8
   <211> 33
   <212> DNA
   <213> Artificial
<220>
   <223> oligonucleotide
<400> 8
   tttttttcta gattaagcgt gcacgttcac gga
   33
<210> 9
   <211> 36
   <212> DNA
   <213> Artificial
<220>
   <223> oligonucleotide
<400> 9
   ttttttgata tcaccggaat tgcagtcatg attggc
   36
<210> 10
   <211> 36
   <212> DNA
   <213> Artificial
<220>
   <223> oligonucleotide
<400> 10
   ttttttgata tcatgtataa tgctgatatg attgac
   36
<210> 11
   <211> 36
   <212> DNA
   <213> Artificial
<220>
   <223> oligonucleotide
<400> 11
   tttttttcta gattaacgtt ttcccgaggc gaagtc
   36
<210> 12
   <211> 42
   <212> DNA
   <213> Artificial
<220>
   <223> oligonucleotide
<400> 12
   atcatcgagc tccagctgta attcatggtc gaaaagaagt gc
   42
<210> 13
   <211> 73
   <212> DNA
   <213> Artificial
<220>
   <223> oligonucleotide
<400> 13
   gaattcctcg agctgcagcc cgggttttta tagctaatta gtcatttttt gagagtacca
   60
   cttcagctac ctc
   73
<210> 14
   <211> 72
   <212> DNA
   <213> Artificial
<220>
   <223> oligonucleotide
<400> 14
   cccgggctgc agctcgagga attcttttta ttgattaact agtcattata aagatctaaa
   60
   atgcataatt tc
   72
<210> 15
   <211> 45
   <212> DNA
   <213> Artificial
<220>
   <223> oligonucleotide
<400> 15
   gatgatggta ccgtaaacaa atataatgaa aagtattcta aacta 45
<210> 16
   <211> 34
   <212> DNA
   <213> Artificial
<220>
   <223> oligonucleotide
<400> 16
   aaacccgggt tctttattct atacttaaaa agtg 34
<210> 17
   <211> 43
   <212> DNA
   <213> Artificial
<220>
   <223> oligonucleotide
<400> 17
   aaaagaattc gtcgactacg atacaaactt aacggatatc gcg 43
<210> 18
   <211> 33
   <212> DNA
   <213> Artificial
<220>
   <223> oligonucleotide
<400> 18
   ttttcgcgaa ccggaattgc agtcatgatt ggc 33
<210> 19
   <211> 39
   <212> DNA
   <213> Artificial
<220>
   <223> oligonucleotide
<400> 19
   ttttgtcgac gcggccgctt aagcgtgcac gttcacgga
   39
<210> 20
   <211> 42
   <212> DNA
   <213> Artificial
<220>
   <223> oligonucleotide
<400> 20
   atcatcgagc tcgcggccgc ctatcaaaag tcttaatgag tt
   42
<210> 21
   <211> 73
   <212> DNA
   <213> Artificial
<220>
   <223> oligonucleotide
<400> 21
   gaattcctcg agctgcagcc cgggttttta tagctaatta gtcatttttt cgtaagtaag
   60
   tatttttatt taa
   73
<210> 22
   <211> 72
   <212> DNA
   <213> Artificial
<220>
   <223> oligonucleotide
<400> 22
   cccgggctgc agctcgagga attcttttta ttgattaact agtcaaatga gtatatataa
   60
   ttgaaaaagt aa
   72
<210> 23
   <211> 45
   <212> DNA
   <213> Artificial
<220>
   <223> oligonucleotide
<400> 23
   gatgatggta ccttcataaa tacaagtttg attaaactta agttg 45
<210> 24
   <211> 33
   <212> DNA
   <213> Artificial
<220>
   <223> oligonucleotide
<400> 24
   aaacccgggc ggtggtttgc gattccgaaa tct 33
<210> 25
   <211> 43
   <212> DNA
   <213> Artificial
<220>
   <223> oligonucleotide
<400> 25
   aaaagaattc ggatccgatt aaacctaaat aattgtactt tgt 43
<210> 26
   <211> 33
   <212> DNA
   <213> Artificial
<220>
   <223> oligonucleotide
<400> 26
   tttcacgtga tgtataatgc tgatatgatt gac 33
<210> 27
   <211> 42
   <212> DNA
   <213> Artificial
<220>
   <223> oligonucleotide
<400> 27
   ttttggatcc gcggccgctt aacgttttcc cgaggcgaag tc
   42
<210> 28
   <211> 39
   <212> DNA
   <213> artificial oligonucleotide
<400> 28
   ttttttgata tcatgaccgg aattgcagtc atgattggc
   39
<210> 29
   <211> 36
   <212> DNA
   <213> artificial oligonucleotide
<400> 29
   ttttcgcgaa tgaccggaat tgcagtcatg attggc
   36

## Description

La présente invention a pour objet des compositions immunogènes et des vaccins contre la fièvre du Nil comprenant un ou plusieurs virus recombinés avipox et exprimant au moins les protéines prM, M et E du virus de la fièvre du Nil.

Sont aussi décrites des méthodes d'immunisation et de vaccination contre ce virus.

Le virus de la fièvre du Nil (West Nile Virus ou WNV) fut identifié pour la première fois en 1937 chez l'homme en Ouganda dans la province de West Nile (Zeller H. G., Med. Trop., 1999, 59, 490-494).

Largement répandu en Afrique, et aussi rencontré en Inde, au Pakistan et dans le bassin méditerranéen, il a été identifié aux USA pour la première fois dans la ville de New York en 1999 (Anderson J. F. et al., Science, 1999, 286, 2331-2333).

Le virus de la fièvre du Nil touche aussi bien les oiseaux que les mammifères et l'homme.

La maladie se caractérise chez les oiseaux par une attaque du système nerveux central et la mort. Les lésions incluent des encéphalites, des hémorragies dans le myocarde et des hémorragies et nécroses dans le tractus intestinal.

Chez les poulets, des infections expérimentales par inoculations sous-cutanées de virus de la fièvre du Nil isolés sur des corneilles ont entraîné des nécroses du myocarde, des néphrites et des pneumonies de 5 à 10 jours après inoculation, des encéphalites, modérées à sévères, 21 jours après inoculation (Senne D. A. et al., Avian Disease, 2000, 44, 642-649).

Le virus de la fièvre du Nil touche aussi les chevaux, notamment en Afrique du Nord et en Europe (Cantile C. et al., Equine Vet. J., 2000, 32(1), 31-35). Ces chevaux montrent des signes d'ataxie, de faiblesse des membres postérieurs, de parésie évoluant vers une tétraplégie et la mort. Les chevaux et les camélidés sont les principaux animaux manifestant des signes cliniques sous forme d'encéphalites.

Des anticorps anti-WNV ont été détectés chez certains rongeurs, chez le bétail, notamment chez des bovins et des ovins, chez des animaux domestiques, notamment chez le chien (Zeller H. G., Med. Trop., 1999, 59, 490-494 ; Lundstrom J. O., Journal of Vector Ecology, 1999. 24(1). 1-39).

L'espèce humaine n'est pas épargnée par le virus de la fièvre du Nil, avec de nombreux symptômes (Sampson B. A., Human Pathology, 2000, 31(5), 527-531 ; Marra C. M., Seminars in Neurology, 2000, 20(3), 323-327).

Le virus de la fièvre du Nil est transmis aux oiseaux et aux mammifères par piqûres de certains moustiques (par exemple *Culex, Aedes, Anopheles*) et de tiques.

Les oiseaux sauvages et domestiques sont un réservoir pour le virus de la fièvre du Nil et un vecteur de propagation par leurs migrations.

Les virions du virus de la fièvre du Nil sont des particules sphériques de 50 nm de diamètre constituées d'une enveloppe lipoprotéique entourant une nucléocapside icosahédrique contenant un ARN simple brin de polarité positive.

Un unique cadre ouvert de lecture (COL) code pour l'ensemble des protéines virales sous la forme d'une polyprotéine. Le clivage et la maturation de cette polyprotéine conduit à la production d'une dizaine de protéines virales différentes. Les protéines structurales sont codées par la partie 5' du génome et correspondent à la nucléocapside nommée C (14 kDa), la glycoprotéine d'enveloppe nommée E (50 kDa), la protéine de pré-membrane nommée prM (23 kDa), la protéine de membrane nommée M (7 kDa). Les protéines non structurales sont codées par la partie 3' du génome et correspondent aux protéines NS1 (40 kDa), NS2A (19 kDa), NS2B (14 kDa), NS3 (74 kDa), NS4A (15 kDa), NS4B (29 kDa), NS5 (97 kDa).

Parrish C. R. et al. (J. Gen. Virol., 1991, 72, 1645-1653), Kulkami A. B. et al. (J. Virol., 1992, 66(6), 3583-3592) et Hill A. B. et al. (J. Gen. Virol., 1992, 73, 1115-1123) ont construit, à partir du virus de la vaccine, des vecteurs d'expression *in vivo* contenant divers inserts correspondant à des séquences nucléotidiques codant pour des protéines non-structurales du virus Kunjin éventuellement associées à des protéines structurales. Ces vecteurs ont été administrés à la souris pour évaluer la réponse immunitaire cellulaire. Les auteurs insistent sur l'importance de la réponse cellulaire, réponse qui est essentiellement stimulée par les protéines non-structurales et en particulier par NS3, NS4A et NS4B. Ces articles font ressortir la difficulté à mettre en évidence quelle serait une bonne stratégie de vaccination contre la fièvre du Nil.

A ce jour, il n'y a pas de vaccin pour prévenir l'infection par le virus WN.

La présente invention a pour objectif de proposer un moyen de prévention et/ou de lutte contre les maladies causées par le virus WN qui puisse être employé chez les équins.

Un autre objectif encore de la divulgation est de proposer des méthodes d'immunisation et de vaccination des espèces cibles.

Un autre objectif de la divulgation est de proposer de tels moyens et méthodes permettant d'assurer un diagnostic différentiel.

### RÉSUMÉ DE L'INVENTION

• Dans son sens le plus large, l'invention a trait à:
1. Une composition immunogène pour une utilisation dans la protection des équins contre le virus de la Fièvre du Nile (WNV) ou vaccin pour une utilisation dans la protection des équins contre le virus de la fièvre du Nile (WN), comprenant un ou plusieurs virus recombinés avipox et exprimant les protéines prM, M et E du virus WN, les virus recombinés comprenant un ou plusieurs polynucléotides codant pour une ou plusieurs protéines prM, M et E, et un véhicule ou excipient pharmaceutiquement acceptable. Selon un mode de réalisation particulier, l'invention a trait à :
2. Composition immunogène ou vaccin pour une utilisation selon 1, caractérisé en ce qu'il comprend un virus recombiné avipox qui exprime les trois protéines prM, M et E.
3. Composition immunogène ou vaccin pour une utilisation selon 1, caractérisé en ce qu'il comporte un virus recombiné avipox qui comprend un polynucléotide formant une phase de lecture codant pour prM-M-E.
4. Composition immunogène ou vaccin selon 1, 2 ou 3, pour une utilisation selon 1, caractérisé en ce que le virus recombiné est un canarypox.
5. Composition immunogène ou vaccin selon 1, 2 ou 3 pour une utilisation selon 1, caractérisé en ce que le virus recombiné est un fowlpox.
6. Composition immunogène ou vaccin selon l'une de 1 à 5 pour une utilisation selon 1, caractérisé en ce que le polynucléotide code aussi pour un peptide signal.
7. Composition immunogène ou vaccin selon 6 pour une utilisation selon 1, caractérisé en ce qu'il s'agit d'un peptide signal du virus WN.
8. Composition immunogène ou vaccin selon 6 pour une utilisation selon 1, caractérisé en ce qu'il s'agit d'un peptide signal hétérologue.
9. Composition immunogène ou vaccin selon 8 pour une utilisation selon 1, caractérisé en ce qu'il s'agit du peptide signal de l'activateur tissulaire du plasminogène humain.
10. Composition immunogène ou vaccin selon l'une quelconque de 1 à 9 , pour une utilisation selon 1 caractérisé en ce qu'il comprend un autre polynucléotide codant pour une protéine d'une autre souche de virus WN et/ou d'un autre agent pathogène et/ou pour une cytokine.
11. Composition immunogène ou vaccin selon l'une quelconque de 1 à 10, pour une utilisation selon 1 caractérisé en ce qu'il comprend un autre vecteur d'expression comprenant un autre polynucléotide codant pour une protéine d'une autre souche de virus WN et/ou d'un autre agent pathogène et/ou pour une cytokine.
12. Composition immunogène ou vaccin selon l'une quelconque de 1 à 11 , pour une utilisation selon 1 caractérisé en ce qu'il comprend en outre un adjuvant.
13. Composition immunogène ou vaccin selon 12 , pour une utilisation selon 1 caractérisé en ce que l'adjuvant est choisi parmi (1) les polymères de l'acide acrylique ou méthacrylique et les polymères d'anhydride maléique et de dérivé alcényle, (2) les séquences immunostimulatrices, (3) une émulsion huile-dans-l'eau, (4) les lipides cationiques contenant un sel d'ammonium quaternaire, (5) les cytokines.
14. Composition immunogène ou vaccin selon 13 , pour une utilisation selon 1 caractérisé en ce que l'adjuvant est un carbomère.
15. Composition immunogène ou vaccin selon 10 ou 13, pour une utilisation selon 1 caractérisé en ce que la cytokine est une cytokine équine ou une cytokine aviaire.
16. Composition immunogène ou vaccin selon 15 , pour une utilisation selon 1 caractérisé en ce que la cytokine est choisie parmi interleukine 18, interleukine 12, interleukine 15, MIP-1a, GM-CSF.
17. Composition immunogène ou vaccin selon 15 , pour une utilisation selon 1 caractérisé en ce que la cytokine est le GM-CSF équin.
18. Composition immunogène ou vaccin selon l'une quelconque de 1 à 17, pour une utilisation selon 1 formant une composition immunogène ou un vaccin multivalent, caractérisé en ce qu'il comprend un composant de composition immunogène ou vaccin contre un autre agent pathogène.
19. Composition immunogène ou vaccin selon 18 , pour une utilisation selon 1 caractérisé en ce que le composant contre un autre agent pathogène comporte de l'antigène sous forme de sous-unité, sous-forme d'agent inactivé ou sous-forme d'agent atténué.
20. Composition immunogène ou vaccin selon 18 ou 19, pour une utilisation selon 1 caractérisé en ce qu'il comprend un composant de composition immunogène ou vaccin contre le virus de la rhinopneumonie équine, contre le virus de la grippe équine, contre le virus de l'encéphalite de l'Est, contre le virus de l'Encéphalite de l'Ouest, contre le virus de l'Encéphalite Vénézuélienne, contre Clostridium tetani, et leurs mélanges.

### DESCRIPTION DETAILLEÉ DE L'INVENTION

La divulgation a pour premier objet les vecteurs d'expression *in vitro* et/ou *in vivo* comprenant un polynucléotide codant pour la protéine d'enveloppe E du virus WN. Ces vecteurs comprennent en plus les éléments nécessaires à l'expression du polynucléotide dans la cellule hôte.

En plus du polynucléotide codant pour E, les vecteurs d'expression Pour utilisation selon l'invention comprennent un ou plusieurs autres polynucléotides codant pour d'autres protéines du virus WN, notamment la protéine de pré-membrane prM et la protéine de membrane M.

Le vecteur comprend de préférence un polynucléotide formant une seule phase codante correspondant e.g. à prM-E, M-E et plus particulièrement à prM-M-E. Un vecteur comprenant plusieurs polynucléotides séparés codant pour les différentes protéines (e.g. prM et/ou M et E) entre aussi dans le cadre de la présente invention. Le vecteur, en particulier *in vivo,* peut aussi comprendre des polynucléotides correspondant à plus d'une souche de virus WN, en particulier deux ou plus polynucléotides codant pour E ou pour prM-M-E de souches différentes. Comme on le verra plus loin, le vecteur, en particulier *in vivo,* peut aussi comprendre une ou des séquences nucléotidiques codant pour des immunogènes d'autres agents pathogènes et/ou pour des cytokines.

Suivant un mode de réalisation préféré, le vecteur d'expression comprend un polynucléotide codant pour prM-M-E et ce de préférence en une seule phase de lecture.

Par polynucléotide codant pour une protéine du virus WN, on entend principalement un fragment d'ADN qui code pour cette protéine, ou le brin complémentaire de ce fragment d'ADN. Un ARN n'est pas exclu.

Au sens de la divulgation, le terme protéine englobe les fragments, y compris peptides et polypeptides. Par définition, le fragment de protéine est immunologiquement actif en ce sens qu'une fois administré à l'hôte, il est susceptible de déclencher une réponse immunitaire de type humorale et/ou cellulaire dirigée contre la protéine. De préférence, le fragment de protéine est tel qu'il possède substantiellement la même activité immunologique que la protéine entière. Un fragment de protéine selon l'invention comprend donc au moins un épitope ou déterminant antigénique. Le terme épitope se rapporte à un site de la protéine capable d'induire une réaction immunitaire de type humorale (cellules B) et/ou de type cellulaire (cellules T).

La structure minimale du polynucléotide est donc celle qui code pour un épitope ou déterminant antigénique de la protéine considérée. Un polynucléotide codant pour un fragment de la protéine totale comprend notamment au minimum 21 nucléotides, notamment au moins 42 nucléotides et de préférence au moins 57, 87 ou 150 nucléotides consécutifs de la séquence dont il est issu. Les techniques de détermination des épitopes sont bien connues de l'homme du métier, on peut notamment utiliser les banques de peptides chevauchants (Hemmer B. et al., Immunology Today, 1998, 19(4), 163-168), Pepscan (Geysen H. M. et al., Proc. Nat. Acad. Sci. USA, 1984, 81(13), 3998-4002 ; Geysen H. M. et al., Proc. Nat. Acad. Sci. USA, 1985, 82(1), 178-182 ; Van der Zee R. et al., Eur. J. Immunol., 1989, 19(1), 43-47 ; Geysen H. M., Southeast Asian J. Trop. Med. Public Health, 1990, 21(4), 523-533 ; Multipin^{®} Peptide Synthesis Kits de Chiron), les algorithmes (De Groot A. et al., Nature Biotechnology, 1999, 17, 533-561).

En particulier les polynucléotides comprennent la séquence nucléotidique codant pour l'un ou les deux domaines transmembranaires, de préférence les deux, situés en partie C terminale de la protéine E. Pour la souche WNV NY99 ces domaines correspondent aux séquences en acides aminés 742 à 766 et 770 à 791 de GenBank AF196835.

Dés éléments nécessaires à l'expression du ou des polynucléotides sont présents, Il s'agita minima d'un codon d'initiation (ATG), d'un codon stop et d'un promoteur, ainsi qu'une séquence de polyadénylation pour les plasmides et les vecteurs viraux autres que poxvirus. Lorsque le polynucléotide code pour un fragment de la polyprotéine, par exemple prM-E, M-E, prM-M-E, un ATG est placé en 5' de la phase de lecture et un codon stop est placé en 3'. Comme il sera expliqué plus loin, d'autres éléments, permettant le contrôle de l'expression, pourront être présents, tels que séquences activatrices (en anglais « enhancer »), séquences stabilisatrices, séquences signal permettant la sécrétion de la protéine.

La présente divulgation a aussi pour objet des préparations comprenant de tels vecteurs d'expression. Plus particulièrement, elle concerne des préparations comprenant un ou plusieurs vecteurs d'expression *in vivo,* comprenant et exprimant un ou plusieurs des polynucléotides ci-dessus, dont celui codant pour E, celui pour prM et celui pour M dans un véhicule ou excipient pharmaceutiquement acceptable.

Suivant une première modalité le ou les autres vecteurs dans la préparation comprennent et expriment une ou plusieurs autres protéines du virus WN, e.g. prM, M, prM-M.

Suivant une autre modalité, le ou les autres vecteurs dans la préparation comprennent et expriment une ou plusieurs protéines d'une ou plusieurs autres souches de virus WN. Notamment, la préparation comprend au moins deux vecteurs exprimant, en particulier *in vivo,* des polynucléotides de souches WN différentes, codant pour les mêmes protéines et/ou pour des protéines différentes, de préférence pour les mêmes protéines. Il s'agit de préférence de vecteurs exprimant *in vivo* E ou prM-M-E de deux, trois ou plus souches WN différentes. La divulgation vise aussi les mélanges de vecteurs exprimant prM, M, E, prM-M, prM-E ou M-E de souches différentes.

Suivant une autre modalité encore, et comme on le verra plus en détail plus loin, le ou les autres vecteurs dans la préparation comprennent et expriment une ou plusieurs cytokines et/ou un ou plusieurs immunogènes d'un ou plusieurs autres agents pathogènes.

La divulgation vise aussi les diverses combinaisons de ces différentes modalités.

Les préparations comprenant un vecteur d'expression *in vitro* ou *in vivo* comprenant et exprimant un polynucléotide codant pour prM-M-E constituent un mode de réalisation préféré de la divulgation

Suivant un mode de réalisation particulier de la divulgation, les vecteurs d'expression *in vivo* ou *in vitro* comprennent comme seul(s) polynucléotide(s) du virus WN, un polynucléotide codant pour la protéine E, associé à prM et/ou M, de préférence codant pour prM-M-E, et éventuellement une séquence signal du virus WN.

Suivant un mode de réalisation particulier, une ou plusieurs des protéines non structurales NS2A, NS2B et NS3 sont exprimées, conjointement aux protéines structurales, soit via le même vecteur d'expression, soit via un vecteur d'expression propre. Elles sont de préférence exprimées ensemble à partir d'un polynucléotide unique.

La divulgation a donc aussi pour objet un vecteur d'expression, *in vivo* ou *in vitro*, comprenant le polynucléotide codant pour NS2A, NS2B, NS3, leurs combinaisons, et de préférence pour NS2A-NS2B-NS3. A la base, ce vecteur peut être l'un des vecteurs décrits ci-dessus, comprenant un polynucléotide codant pour une ou des protéines structurales, notamment E ou prM-M-E. En variante, la divulgation concerne une préparation telle que décrite ci-dessus, comportant en outre au moins un de ces vecteurs exprimant une protéine non structurale et éventuellement un véhicule ou excipient pharmaceutiquement acceptable.

Pour réaliser les vecteurs d'expression l'homme du métier dispose de différentes souches du virus WN et de la description de la séquence nucléotidique de leur génome. Voir notamment Savage H. M. et al. (Am. J. Trop. Med. Hyg. 1999, 61(4), 600-611), tableau 2, qui mentionne 24 souches de virus WN et donne les références d'accès à des séquences polynucléotidiques dans GenBank.

On peut par exemple se référer à la souche NY99 (GenBank AF196835). Dans GenBank, il est précisé pour chaque protéine la séquence d'ADN correspondante (nucléotides 466-741 pour prM, 742-966 pour M, 967-2469 pour E, soit 466-2469 pour prM-M-E, 3526-4218 pour NS2A, 4219-4611 pour NS2B et 4612-6468 pour NS3, soit 3526-6468 pour NS2A-NS2B-NS3). Par comparaison et alignement de séquences, la détermination d'un polynucléotide codant pour telle protéine dans une autre souche de WNV est immédiate.

Il a été indiqué plus haut que par polynucléotide, on entendait la séquence codant pour la protéine ou un fragment ou un épitope, spécifique du virus WN. En outre, par équivalence, le terme polynucléotide englobe aussi les séquences nucléotidiques correspondantes des différentes souches de virus WN et les séquences nucléotidiques qui diffèrent par la dégénérescence du code.

Au sein de la famille des virus WN, l'identité entre séquences en acides aminés prM-M-E par rapport à celle de NY99 est égale ou supérieure à 90 %. Sont donc comprises dans la divulgation les polynucléotides codant pour une séquence d'acides aminés dont l'identité avec la séquence en acides aminés native est supérieure ou égale à 90 %, notamment à 92 %, de préférence à 95 %, et plus particulièrement encore 98 %. Seront aussi considérés comme des équivalents les fragments de ces polynucléotides homologues, qui sont spécifiques des virus WN.

Ainsi, si l'on parle d'un polynucléotide du virus WN, ce terme englobe les séquences équivalentes au sens de l'invention.

On a vu aussi que le terme protéine englobe les polypeptides et peptides immunologiquement actifs. Pour les besoins de l'invention, ceci englobe :
a) les protéines correspondantes des différentes souches de virus WN;
b) les protéines qui en diffèrent mais qui conservent avec une protéine native WN une identité supérieure ou égale à 90 %, notamment à 92 %, de préférence à 95%, et plus particulièrement encore 98 %.

Ainsi, si l'on parle d'une protéine du virus WN, ce terme englobe les protéines équivalentes au sens de l'invention.

Différentes souches de virus WN sont accessibles auprès de collection, notamment auprès de l'American Type Culture Collection (ATCC), par exemple sous les numéros d'accès VR-82 ou VR-1267. Le virus dénommé Kunjin est considéré, comme étant en fait un virus WN.

On préfère que le polynucléotide comprenne en plus une séquence nucléotidique codant pour un peptide signal, situé en amont de la protéine exprimée pour en assurer la sécrétion. Il peut donc s'agir d'une séquence endogène, c'est-à-dire de la séquence signal naturelle lorsqu'elle existe (provenant du même virus WN ou d'une autre souche). Par exemple, pour le virus WN NY99, la séquence signal endogène de E correspond aux nucléotides 922 à 966 de la séquence GenBank ; pour prM, il s'agit des nucléotides 421 à 465. Il peut aussi s'agir d'une séquence nucléotidique codant pour un peptide signal hétérologue, notamment celle codant pour le peptide signal de l'activateur, tissulaire du plasminogène humain (tPA) (Hartikka J. et al., Human Gene Therapy, 1996, 7, 1205-1217). La séquence nucléotidique codant pour le peptide signal est insérée en phase et en amont de la séquence codant pour E ou ses combinaisons, e.g. prM-M-E.

Les vecteurs d'expression *in vivo* sont des vecteurs viraux, les avipox (en particulier canarypox, fowlpox, pigeonpox, quailpox

Le vecteur d'expression est un canarypox ou un fowlpox, ces poxvirus pouvant être éventuellement atténués. On peut citer le canarypox commercialement disponible auprès de l'ATCC sous le numéro d'accès VR-111. Des canarypox atténués ont été décrits dans US-A-5;756,103 et dans WO-A-01/05934. De nombreuses souches vaccinales de virus fowlpox sont accessibles, par exemple le vaccin DIFTOSEC CT® commercialisé par MERIAL et le vaccin NOBILS® VARIOLE commercialisé par Intervet.

Pour les poxvirus, l'homme du métier peut se reporter à WO-A-90/12882, pour le fowlpox à US-A-5,174,993 ; US-A-5,505,941 ; US-5,766,599 ; pour le canarypox à US-A-5,756,103 ; pour le swinepox à US-A-5, 382, 425 ; pour le raccoonpox à WO-A-00/03030.

Les sites d'insertion du (des) polynucléotide(s) à exprimer sont en particulier situés dans, ou constitués par, les COLs C3, C5 et C6. Les sites d'insertion sont en particulier situés dans, ou constitués par, les COLs F7 et F8.

De préférence, le polynucléotide à exprimer est inséré sous le contrôle d'un promoteur spécifique des poxvirus, notamment le promoteur vaccine 7,5 kDa (Cochran et al., J. Virology, 1985, 54, 30-35), le promoteur vaccine I3L (Riviere et al., J. Virology, 1992, 66, 3424-3434), le promoteur vaccine HA (Shida, Virology, 1986, 150, 451-457), le promoteur cowpox ATI (Funahashi et al., J. Gen. Virol., 1988, 69, 35-47), ou encore le promoteur vaccine H6 (Taylor J. et al. Vacciné, 1988, 6, 504-508 ; Guo P. et al. J. Virol., 1989, 63, 4189-4198 ; Perkus M. et al. J. Virol., 1989, 63, 3829-3836).

De préférence le vecteur d'expression est un canarypox.

Sont également décrits des vecteurs d'expression utilisés pour exprimer *in vitro* les protéines dans un système cellulaire approprié. Les protéines peuvent être ensuite récoltées dans le surnageant de culture, après sécrétion ou non (s'il n'y a pas sécrétion, on procède à une lyse cellulaire), éventuellement concentrées par des techniques classiques de concentration, notamment par ultrafiltration et/ou purifiées par des moyens de purification classiques, notamment les techniques de chromatographie de type affinité, échange d'ion, ou gelfiltration.

La production est effectuée par transfection de cellules de mammifère par des plasmides, par réplication dé vecteurs viraux sur cellules de mammifère ou cellules aviaires, ou par réplication de Baculovirus (US-A-4 745 051 ; Vialard J. et al., J. Virol., 1990, 64(1), 37-50 ; Verne A., Virology, 1988, 167, 56-71), e.g. Autographa califôrnica Nuclear Polyhedrosis Virus AcNPV, sur cellules d'insectes (par exemple cellules Sf9 *Spodoptera frugiperda*, dépôt ATCC CRL 1711). Comme cellules de mammifères on peut utiliser notamment des cellules de hamster (par exemple CHO ou BHK-21), des cellules de singe (par exemple COS ou VERO). La divulgation couvre donc également ces vecteurs d'expression incorporant un polynucléotide selon la divulgation les protéines de WN ou fragments ainsi produits et les, préparations les contenant.

La présente divulgation, a donc aussi pour objet les préparations purifiées et/ou concentrées de protéines WN. Lorsque le polynucléotide code pour plusieurs protéines celles-ci sont clivées, et les préparations ci-dessus contiennent alors des protéines clivées.

L'invention a pour objet les compositions immunogènes et les vaccins contre le virus WN tels que décrits dans les revendications pour une utilisation telle que décrite dans les revendications.

La notion de composition immunogène recouvre toute composition susceptible, une fois administrée à l'espèce cible, d'induire une réponse immunitaire dirigée contre le virus WN. Par vaccin, on entend une composition capable d'induire une protection efficace. Les espèces cibles sont les équins.

Les véhicules ou excipients pharmaceutiquement acceptables sont parfaitement connus de l'homme du métier. A titre d'exemple, il peut s'agir de solution saline NaCl à 0,9% ou de tampon phosphate. Les véhicules ou excipients pharmaceutiquement acceptables englobent aussi tout composé ou combinaison de composés permettant la facilitation de l'administration du vecteur, notamment de la transfection, et/ou l'amélioration de la conservation.

Les doses et volumes de dose sont définis plus loin dans le cadre de la description générale des méthodes d'immunisation et de vaccination.

Les compositions immunogènes et les vaccins pour utilisation selon l'invention comprennent de préférence un ou plusieurs adjuvants, choisis notamment parmi les adjuvants usuels. Conviennent particulièrement bien dans le cadre de la présente invention : (1) polymères de l'acide acrylique ou méthacrylique, polymères d'anhydride maléique et de dérivé alcényle, (2) séquences immunostimulatrices (ISS), notamment séquences oligodésoxyribonucléotidiques ayant un ou plusieurs motifs CpG non méthylés (Klinman D. M. et al., Proc. Natl. Acad. Sci. USA, 1996, 93, 2879-2883 ; WO-A1-98/16247), (3) une émulsion huile-dans-l'eau, en particulier l'émulsion SPT décrite à la page 147 de « Vaccine Design, The Subunit and Adjuvant Approach » edited by M. Powell, M. Newman, Plenum Press 1995, et l'émulsion MF59 décrite à la page 183 du même ouvrage, (4) lipides cationiques contenant un sel d'ammonium quaternaire, (5) cytokines, ou (6) leurs combinaisons ou mélanges.

L'émulsion huile-dans-l'eau (3), qui est particulièrement adaptée pour les vecteurs viraux, peut notamment être à base :
- d'huile de paraffine liquide légère (type Pharmacopée européenne) ;
- d'huile isoprénoide telle que le squalane, le squalène ;
- d'huile résultant de l'oligomérisation d'alcènes, en particulier d'isobutène ou de decène ;
- d'esters d'acides ou d'alcools à groupement alkyle linéaire ;
- plus particulièrement huiles végétales, oléate d'éthyle, di(caprylate / caprate) de propylène glycol, tri(caprylate / caprate) de glycérol, dioléate de propylène glycol ;
d'esters d'acides ou d'alcools gras ramifiés, en particulier esters de l'acide isostéarique.

L'huile est utilisée en association avec des émulsifiants pour former l'émulsion. Les émulsifiants sont de préférence des tensio-actifs non ioniques, en particulier :
- les esters d'une part de sorbitan, de mannide (e.g. oléate d'anhydromannitol), de glycérol, de polyglycérol, ou de propylène glycol et d'autre part d'acide oléique, isostéarique, ricinoléique, hydroxystéarique, ces esters étant éventuellement éthoxylés,
- les blocs copolymères polyoxypropylène-polyoxyéthylène, en particulier les Pluronic®, notamment L121.

Parmi les polymères adjuvants de type (1), on préfère les polymères de l'acide acrylique ou méthacrylique réticulés, notamment réticulés par des éthers polyalcényliques de sucres ou de polyalcools. Ces composés sont connus sous le terme carbomère (Pharmeuropa vol. 8, n° 2, juin 1996). L'homme de l'art peut aussi se référer à US-A-2 909 462 qui décrit de tels polymères acryliques réticulés par un composé polyhydroxylé ayant au moins 3 groupes hydroxyle, de préférence pas plus de 8, les atomes d'hydrogène d'au moins trois hydroxyles étant remplacés par des radicaux aliphatiques insaturés ayant au moins 2 atomes de carbone. Les radicaux préférés sont ceux contenant de 2 à 4 atomes de carbone, e.g. vinyles, allyles et autres groupes éthyléniquement insaturés. Les radicaux insaturés peuvent eux-mêmes contenir d'autres substituant, tels que méthyl. Les produits vendus sous la dénomination Carbopol® (BF Goodrich, Ohio, USA) sont particulièrement appropriés. Ils sont notamment réticulés par un allyl saccharose ou par de l'allylpentaérythritol. Parmi eux, on peut citer en particulier les Carbopol® 974P, 934P et 971P.

Parmi les copolymères d'anhydride maléique et de dérivé alcényle, on préfère les EMA® (Monsanto) qui sont des copolymères d'anhydride maléique et d'éthylène, linéaires ou réticulés, par exemple réticulés par du divinyléther. On peut se référer à J. Fields et al., Nature, 186: 778-780,4 juin 1960.

Sur le plan de leur structure, les polymères d'acide acrylique ou méthacrylique et les EMA® sont formés de préférence de motifs de base de formule suivante : 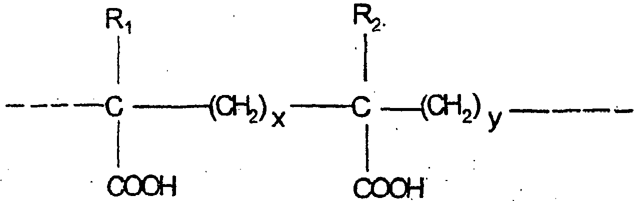 dans laquelle:
- R₁ et R₂, identiques ou différents, représentent H ou CH₃
- x = 0 ou 1, de préférence x = 1
- y = 1 ou 2, avec x + y = 2

Pour les EMA®, x = 0 et y = 2. Pour les carbomères, x = y = 1.

Ces polymères sont dissous dans l'eau ou dans de l'eau physiologique (NaCl à 20 g/l) et le pH est ajusté à 7,3-7,4 par de la soude, pour donner la solution adjuvante dans laquelle les vecteurs d'expression seront incorporés.

La concentration en polymère dans la composition vaccinale finale peut notamment aller de 0,01 % à 1,5 %:P/V, plus particulièrement de 0,05 à 1 % PN, de préférence de 0,1 à 0,4 % P/V.

Les lipides cationiques (4) contenant un sel d'ammonium quaternaire, qui sont particulièrement mais pas exclusivement adaptés pour les plasmides, sont dé préférence ceux qui répondent à la formule suivante : 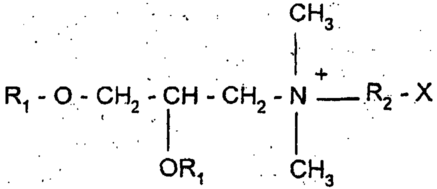 dans laquelle R1 est un radical aliphatique linéaire, saturé ou insaturé, ayant 12 à 18 atomes de carbone, R2 est un autre radical aliphatique, renfermant 2 ou 3 atomes de carbone, et X un groupement hydroxyle ou amine.

Parmi ces lipides cationiques, on préfère le DMRIE (N-(2-hydroxyéthyl)-N,N=diméthyl-2,3-bis(tetradécyloxy)-1-propanammonium; WO-A-96/34109), de préférence associé avec un lipide neutre, de préférence le DOPE (dioléoyl-phosphatidyl-éthanolamine; Behr J. P., 1994, Bioconjugate Chemistry, 5, 382-389), pour former le DMRIE-DOPE.

De préférence, le mélange plasmide avec cet adjuvant se fait de manière extemporanée et l'on préfère, avant son administration, laisser le temps au mélange ainsi constitué de se complexer, par exemple pendant une durée allant de 10 à 60 minutes, notamment de l'ordre de 30 minutes.

Lorsque du DOPE est présent, le ratio molaire DMRIE : DOPE va de préférence de 95 : 5 à 5 : 95, plus particulièrement de 1 : 1.

Le ratio pondéral plasmide : adjuvant DMRIE ou DMRIE-DOPE peut aller notamment de 50 : 1 à 1 : 10, en particulier de 10 : 1 à 1 : 5, et de préférence de 1 : 1 à 1 : 2.

La ou les cytokines (5) peuvent être apportée(s) sous forme de protéine à la composition ou vaccin, ou être co-exprimée(s) chez l'hôte avec le ou les immunogènes. La préférence va à la co-expression de la ou des cytokines, soit par le même vecteur que celui exprimant l'immunogène, soit par un vecteur propre.

Les cytokines peuvent notamment être choisies parmi : interleukine 18 (IL-18), interleukine 12 (IL-12), interleukine 15 (IL-15), MIP-1α (macrophage inflammatory protein 1α ; Marshall E. et al., Br. J. Cancer, 1997, 75(12), 1715-1720), GM-CSF (facteur de stimulation de la formation de colonies granulo-macrophagiques, ou en anglais Granulocyte-Macrophage Colony-Stimulating Factor)). On peut citer en particulier des cytokines aviaires, notamment celles du poulet, telles que clL-18 (Schneider K. et al., J. Interferon Cytokine Res., 2000, 20(10), 879-883), clL-15 (Xin K.-Q. et al., Vaccine, 1999, 17, 858-866), et des cytokines équines, notamment le GM-CSF équin (WO-A-00/77210)). De manière préférée, on utilise les cytokines de l'espèce à vacciner.

WO-A-00/77210 décrit la séquence nucléotidique et la séquence en acides aminés correspondant au GM-CSF équin, la production de GM-CSF *in vitro* et la construction de vecteurs (plasmides et vecteurs viraux) permettant, l'expression du GM=CSF équin *in vivo.* Ces protéine, plasmides et vecteurs viraux, peuvent être utilisés dans les compositions immunogènes et les vaccins équins pour utilisation selon l'invention. A titre d'exemple, on peut utiliser le plasmide pJP097 décrit à l'exemple 3 de cette demande antérieure ou utiliser l'enseignement de cette demande antérieure pour produire d'autres vecteurs ou pour produire *in vitro* du GM-CSF équin, et incorporer ces vecteurs ou ce GM-CSF équin dans les compositions immunogènes ou vaccins équins pour utilisation selon l'invention.

Le présent document décrit des compositions immunogènes et des vaccins dits de sous-unités, comprenant la protéine E, et éventuellement une ou plusieurs autres protéines du virus WN, notamment prM ou M, de préférence produits par expression *in vitro* comme décrit ci-dessus, et d'autre part un véhicule ou excipient pharmaceutiquement acceptable.

Les véhicules ou excipients pharmaceutiquement acceptables sont parfaitement connus de l'homme du métier. A titre d'exemple, il peut s'agir de solution saline NaCl à 0,9% ou de tampon phosphate.

Les compositions immunogènes et les vaccins de sous-unités décrits comprennent de préférence un ou plusieurs adjuvants, choisis notamment parmi les adjuvants usuels. Conviennent particulièrement bien : (1) un polymère de l'acide acrylique ou méthacrylique, un polymère d'anhydride malëique et de dérivé alcényle, (2) une séquence immunostimulatrice (ISS), notamment une séquence oligodésoxyribonucléotidique ayant un ou plusieurs motifs CpG non méthylés (Klinman D. M. et al., Proc. Natl. Acad. Sci. USA, 1996, 93, 2879-2883 ; WO-A1-98/16247), (3) une émulsion huile-dans-l'eau en particulier l'émulsion SPT décrite à la page 147 de « Vaccine Design, The Subunit and Adjuvant Approach » edited by M. Powell, M. Newman, Plénum Press 1995, et l'émulsion MF59 décrite à la page 183 du même ouvrage, (4) une émulsion eau-dans-l'huile (EP-A-639 071), (5) la saponine, en particulier Quil-A, ou (6) de l'hydroxyde d'alumine ou équivalent. Les différents types d'adjuvants définis sous 1), 2) et 3) ont été décrits précédemment plus en détail en regard des vaccins à base de vecteurs d'expression.

Les doses et volumes de dose sont définis plus loin dans le cadre de la description générale des méthodes d'immunisation et de vaccination.

Conformément à l'invention, la vaccination contre le virus WN peut être associée à d'autres vaccinations dans le cadre de programmes de vaccination, sous forme de kits d'immunisation ou de vaccination ou encore sous forme de compositions immunogènes et de vaccins multivalents, c'est-à-dire comprenant au moins un composant de vaccin contre le virus WN et au moins un composant de vaccin contre au moins un autre agent pathogène. Ceci inclut aussi l'expression par un même vecteur d'expression de gènes d'au moins deux agents pathogènes, virus WN inclus.

L'invention a ainsi pour objet une composition immunogènes multivalente ou un vaccin multivalent pour une utilisation dans la protection des équins contre le virus W.N et contre au moins un autre pathogène de l'espèce cible, utilisant un même vecteur d'expression *in vivo* contenant et exprimant les protéines prM, M et E du virus WN et au moins un polynucléotide exprimant un immunogène d'un autre pathogène, comprenant un ou plusieurs virus recombinés avipox.

Par «immunogène» ainsi exprimé, on entend notamment une protéine, glycoprotéine, polypeptide ou peptide, épitope ou dérivé, e.g. protéine de fusion, induisant une réponse immunitaire, de préférence protectrice.

Comme cela a été décrit précédemment, ces compositions ou vaccins multivalents comprennent aussi un véhicule ou excipient pharmaceutiquement acceptable, et éventuellement un adjuvant.

L'invention a également pour objet une composition immunogène multivalente ou un vaccin multivalent comprenant au moins un vecteur d'expression *in vivo* de type virus recombiné avipox exprimant les protéines prM, M et E du virus WN, dans lequel est inséré au moins un polynucléotide du virus WN et au moins un deuxième vecteur d'expression dans lequel est inséré un polynucléotide codant pour un immunogène d'un autre agent pathogène pour une utilisation dans la protection des équins contre le virus WN et contre au moins un autre pathogène de l'espèce cible. Comme cela a été décrit précédemment, ces compositions ou vaccins multivalents comprennent aussi un véhicule ou excipient pharmaceutiquement acceptable, et éventuellement un adjuvant.

Pour les compositions immunogènes et les vaccins multivalents, lesdits autres pathogènes équins sont notamment choisis parmi le groupe comprenant les virus de la rhinopneumonie équine EHV-1 et/ou EHV-4 (de préférence on associe des immunogènes de EHV-1 et EHV-4), virus de la grippe équine EIV, virus de l'encéphalite de l'Est EEV, virus de l'encéphalite de l'Ouest WEV, virus de l'encéphalite Vénézuélienne VEV (on préfère une combinaison des trois EEV, WEV et VEV), *Clostridium tétani* (tétanos), et leurs mélanges. De préférence, pour EHV on choisit les gènes gB et/ou gD ; pour EIV les gènes HA, NP et/ou N ; pour les virus des encéphalites C et/ou E2 ; pour *Clostridium tetani* le gène codant pour tout ou partie de la sous-unité C de la toxine tétanique. Ceci inclut l'utilisation de polynucléotides codant pour un fragment immunologiquement actif ou un épitope de cet immunogène.

A titre d'exemple, dans une composition immunogène multivalente ou un vaccin multivalent selon l'invention, éventuellement adjuvé comme décrit ci-dessus, destiné à l'espèce équine, on peut incorporer un ou plusieurs des plasmides décrits dans WO-A-98/03198 et en particulier dans les exemples 8 à 25 de cette demande antérieure, et ceux décrits dans WO-A-00/77043 et qui concernent l'espèce équine, en particulier ceux décrits dans les exemples 6 et 7 de cette demande antérieure.

Les compositions immunogènes ou les vaccins recombinés tels que décrits précédemment peuvent également être associés avec au moins un vaccin classique (inactivé, vivant atténué, sous-unités) dirigé contre au moins un autre pathogène.

La présente invention décrit aussi des méthodes d'immunisation et de vaccination des espèces cibles mentionnées ci-dessus.

Ces méthodes comprennent l'administration d'une quantité efficace d'une composition immunogène ou d'un vaccin selon l'invention. Cette administration peut se faire notamment par la voie parentérale, e.g. par administration sous-cutanée, intradermique, intramusculaire, ou par la voie orale et/ou nasale. Une ou plusieurs administrations peuvent être réalisées, notamment deux.

Les différents vaccins peuvent être injectés par un injecteur à jet liquide sans aiguille. Pour les plasmides on peut aussi utiliser les particules d'or enrobées de plasmide et projetées de façon à pénétrer dans les cellules de la peau du sujet à immuniser (Tang et al. Nature 1992. 356. 152-154).

Les compositions immunogènes et les vaccins pour utilisation selon l'invention comprennent une quantité efficace de vecteur d'expression ou de polypeptide.

Dans le cas des compositions immunogènes ou des vaccins à base de plasmide, une dose comporte de manière générale de 10 µg environ à 2000 µg environ, notamment de 50 µg environ à 1000 µg environ. Les volumes de dose peuvent être compris entre 0,1 et 2 ml, de préférence entre 0,2 et 1 ml.

Ces doses et volumes de dose sont bien adaptés pour la vaccination des équins.

Dans le cas des compositions immunogènes ou des vaccins à base de poxvirus, une dose est de manière générale comprise entre environ 10² pfu et environ 10⁹ pfu.

Les volumes de dose des compositions immunogènes et des vaccins équins à base de vecteurs viraux sont en général compris entre 0,5 et 2,0 ml, de préférence 1,0 entre et 2,0 ml, préférentiellement de 1,0 ml.

L'homme de l'art possède aussi pour ce type de vaccin les compétences nécessaires pour optimiser le nombre d'administrations, la voie d'administration et les doses à utiliser pour chaque protocole d'immunisation. En particulier on prévoit deux administrations chez le cheval, par exemple à 35 jours d'intervalle.

La divulgation concerne aussi l'utilisation d'un vecteur d'expression in vivo ou d'une préparation de vecteurs ou de polypeptides selon la divulgation pour la préparation d'une composition immunogène ou d'un vaccin destiné à protéger les espèces cibles contre le virus WN et éventuellement contre au moins un autre agent pathogène. Les différentes caractéristiques énoncées dans le reste de la description s'appliquent à cet autre objet de la divulgation

Un vaccin viral exprimant une ou plusieurs protéines du virus WN pour utilisation selon la présente invention, n'induira pas chez l'animal vacciné la production d'anticorps contre les autres protéines de ce virus qui ne sont pas représentées dans la composition immunogène ou vaccin. Cette caractéristique peut être utilisée pour le développement de méthodes de diagnostic différentiel permettant de faire la distinction entre les animaux infectés par le virus pathogène WN et les animaux vaccinés à l'aide de vaccins selon l'invention. Chez ces derniers, ces protéines et/ou des anticorps dirigés contre elles sont présents et peuvent être détectés par une réaction antigène-anticorps. Ce n'est pas le cas chez les animaux vaccinés conformément à l'invention qui restent négatifs. Pour réaliser cette discrimination, on utilise une protéine qui n'est pas représentée dans le vaccin (non présente ou non exprimée), par exemple la protéine C ou la protéine NS1, NS2A, NS2B ou NS3 lorsqu'elle n'est pas représentée dans le vaccin.

La présente divulgation décrit donc l'utilisation des vecteurs, préparations et polypeptides selon la divulgation pour la préparation de compositions immunogènes et de vaccins selon l'intention permettant de discriminer entre animaux vaccinés et animaux infectés.

Elle décrit aussi une méthode d'immunisation et de vaccination associée à une méthode de diagnostic permettant cette discrimination.

La protéine sélectionnée pour le diagnostic ou l'un de ses fragments ou épitopes est utilisée comme antigène dans le test de diagnostic, et/ou est utilisée pour produire des anticorps, polyclonaux ou monoclonaux. L'homme du métier dispose des connaissances et de la pratique pour produire ces anticorps et pour mettre en oeuvre antigènes et/ou anticorps dans les techniques de diagnostic classiques, e.g. tests ELISA.

L'invention va être maintenant décrite plus en détail à l'aide de modes de réalisation pris à titre d'exemples non limitatifs.

### Exemples :

Toutes les constructions sont réalisées en utilisant les techniques standards de biologie moléculaire (clonage, digestion par les enzymes de restriction, synthèse d'un ADN complémentaire simple brin, amplification en chaîne par polymérase, élongation d'un oligonucléotide par une ADN polymérase...) décrites par Sambrook J. et al. (Molecular Cloning: A Laboratory Manual. 2nd Edition. Cold Spring Harbor Laboratory. Cold Spring Harbor. New York. 1989). Tous les fragments de restriction utilisés pour la présente invention, ainsi que les divers fragments d'amplification en chaîne par polymérase (= ACP ou PCR), sont isolés et purifiés en utilisant le kit "Geneclean®" (BIO101 Inc. La Jolla, CA).

### Exemple 1 : Culture du virus de la fièvre du Nil

Pour leur amplification, des virus de la fièvre du Nil NY99 (Lanciotti R. S. et al., Science, 1999, 286, 2333-7) sont cultivées sur cellules VERO (cellules rénales de singe, accessible auprès de l'American Type Culture Collection (ATCC) sous le numéro CCL-81).

Les cellules VERO sont mises en culture en Falcon 25 cm² avec du milieu Eagle-MEM supplémenté de 1 % d'extraits de levure et de 10 % de sérum de veau contenant environ 100 000 cellules par ml. Les cellules sont cultivées à +37°C, sous atmosphère à 5 % de CO₂.

Après 3 jours la couche cellulaire arrive à confluence. Le milieu de culture est alors remplacé par du milieu Eagle-MEM supplémenté de 1 % d'extraits de levure et de 0,1 % d'albumine de sérum de boeuf et le virus de la fièvre du Nil est ajouté à raison de 5 pfu/cellule.

Lorsque l'effet cytopathogène (CPE) est complet (généralement 48-72 heures après le début de la mise en culture), les suspensions virales sont récoltées, puis clarifiées par centrifugation et congelées à -70°C. 3 à 4 passages successifs sont généralement nécessaires à la production d'un lot viral. Le lot viral est stocké à -70°C.

### Exemple 2 : Extraction de l'ARN viral du virus de la fièvre du Nil

L'ARN viral contenu dans 100 ml de suspension virale de la souche de virus de la fièvre du Nil NY99 est extrait après décongélation avec les solutions du kit « High Pure™ Viral RNA Kit » Cat # 1 858 882, Roche Molecular Biochemicals), en suivant les instructions du fournisseur pour les étapes d'extraction. Le culot d'ARN obtenu à la fin de l'extraction est resuspendu avec 1 à 2 ml d'eau distillée stérile sans RNase.

### Exemple 3 : Construction du plasmide pFC101

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple: 2) et avec les oligonucléotides suivants :
FC101 (30 mer) (SEQ ID NO :1)
5'TTTTTTGAATTCGTTACCCTCTCTAACTTC 3'
et FC102 (33 mer) (SEQ ID NO :2)
5'TTTTTTTCTAGATTACCTCCGACTGCGTCTTGA 3'.

Ce couple d'oligonucléotides permet l'incorporation d'un site de restriction EcoRI et d'un site de restriction Xbal et d'un codon stop en 3' de l'insert.

La synthèse du premier brin d'ADNc se fait par élongation de l'oligonucléotide FC102, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC101 et FC102 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 302 pb.

Ce fragment est digéré par EcoRI puis par XbaI pour isoler, auprès électrophorèse en gel d'agarose, le fragment EcoRI-XbaI d'environ 290 pb. Ce fragment est appelé fragment A.

Le plasmide d'expression eucaryote pVR1020 (C. J. Luke et al. J. of Infectious Diseases. 1997. 175. 95-97), dérivé du plasmide pVR1012 (figure 1 et exemple 7 de WO-A-98/03199; Hartikka J. et al., 1997, Human Gene Therapy, 7, 1205-1217), contient la phase codante de la séquence-signal de l'activateur du plasminogène tissulaire humain (tPA).

Un plasmide pVR1020 est modifié par digestion BamHI-BglII et insertion d'une séquence contenant plusieurs sites de clonage (BAMHI, NotI, EcoRI, XbaI, PmlI, PstI, BglII) et résultant de l'appariement des oligonucléotides suivants:
PB326 (40 mer) (SEQ ID NO :3)
5'GATCTGCAGCACGTGTCTAGAGGATATCGAATTCGCGGCC 3' et
PB329 (40 mer) (SEQ ID NO:4)
5' GATCCGCGGGCGCGAATTCGATATCCTCTAGACACGTGCT 3'.

Le vecteur ainsi obtenu, d'une taille d'environ 5105 paires de bases (ou pb), est nommé pAB110.

Le fragment A est ligaturé avec le plasmide d'expression pAB110 préalablement digéré par XbaI et EcoRI, pour donner le plasmide pFC101 (5376 pb). Ce plasmide contient, sous le contrôle du promoteur précoce du cytomégalovirus humain ou hCMV-IE (human Cytomegalovirus Immediate Early), un insert codant pour la séquence-signal de l'activateur du tPA suivie de la séquence codant pour la protéine prM.

### Exemple 4 : Construction du plasmide pFC102

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple 2) et avec les oligonucléotides suivants :
FC103 (30 mer) (SEQ ID NO :5)
5' TTTTTTGAATTCTCACTGACAGTGCAGACA 3'
et FC104 (33 mer) (SEQ ID NO :6)
5' TTTTTTTCTAGATTAGCTGTAAGCTGGGGCCAC 3'.

Ce couple d'oligonucléotides permet l'incorporation d'un site de restriction EcoRI et d'un site de restriction XbaI et d'un codon stop en 3' de l'insert.

La synthèse du premier brin d'ADNc se fait par élongation de l'oligonucléotide FC104, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC103 et FC104 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 252 pb.

Ce fragment est digéré par EcoRI puis par XbaI pour insoler, après électrophorèse en gel d'agarose, le fragment EcoRI-XbaI d'environ 240 pb. Ce fragment est ligaturé avec le plasmide d'expression pAB110 (exemple 3) préalablement digéré par XbaI et EcoRI, pour donner le plasmide pFC102 (5326 pb). Ce plasmide contient, sous le contrôle du promoteur précoce du cytomégalovirus humain ou hCMV-IE (human Cytomegalovirus Immediate Early), un insert codant pour la séquence-signal de l'activateur du tPA suivie de la séquence codant pour la protéine M.

### Exemple 5 : Construction du plasmide pFC103

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple 2) et avec les oligonucléotides suivants :
FC105 (30 mer) (SEQ ID NO :7)
5' TTTTTTGAATTCTTCAACTGCCTTGGAATG 3'
et FC106 (33 mer) (SEQ ID NO :8)
5' TTTTTTTCTAGATTAAGCGTGCACGTTGACGGA 3'.

Ce couple d'oligonucléotides permet l'incorporation d'un site de restriction EcoRI et d'un site de restriction XbaI et d'un codon stop en 3' de l'insert.

La synthèse du premier brin d'ADNc se fait par élongation de l'oligonucléotide FC106, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC105 et FC106 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 1530 pb.

Ce fragment est digéré par EcoRI puis par XbaI pour isoler, après électrophorèse en gel d'agarose, le fragment EcoRI-XbaI d'environ 1518 pb. Ce fragment est ligaturé avec le plasmide d'expression pAB110 (exemple 3) préalablement digéré par XbaI et EcoRI, pour donner le plasmide pFC103 (6604 pb). Ce plasmide continent, sous le contrôle du promoteur précoce du cytomégalovirus humain ou hCMV-IE (human Cytomegalovirus Immediate Early), un insert codant pour la séquence-signal de l'activateur du tPA suivie de la séquence codant pour la protéine E.

### Exemple 6 : Construction du plasmide pFC104

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple 2) et avec les oligonucléotides suivants :
FC101 (30 mer) (SEQ ID NO:1)
et FC106 (33 mer) (SEQ ID NO :8).

Ce couple d'oligonucléotides permet l'incorporation d'un site de restriction EcoRI et d'un site de restriction XbaI et d'un codon stop en 3' de l'insert.

La synthèse du premier brin d'ADNc se fait par élongation de l'oligonucléotide FC106, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC101 et FC106 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 2031 pb.

Ce fragment est digéré par EcoRI puis par XbaI pour isoler, après électrophorèse en gel d'agarose, le fragment EcoRI-XbaI d'environ 2019 pb. Ce fragment est ligaturé avec le plasmide d'expression pAB110 (exemple 3) préalablement digéré par XbaI et EcoRI, pour donner le plasmide pFC104 (7105 pb). Ce plasmide contient, sous le contrôle du promoteur précoce du cytomégalovirus humain ou hCMV-IE (human Cytomegalovirus Immediate Early), un insert codant pour la séquence-signal de l'activateur du tPA suivie de la séquence codant pour la protéine prM-M-E.

### Exemple 7 : Construction du plasmide pFC105

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT.06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple 2) et avec les oligonucléotides suivants:
FC107 (36 mer) (SEQ ID NO :9)
5' TTTTTTGATATCACCGGAATTGCAGTCATGATTGGC 3'
et FC106 (33 mer) (SEQ ID NO :8).

Ce couple d'oligonucléotides permet l'incorporation d'un site de restriction EcoRV et d'un site de restriction XbaI et d'un codon stop en 3' de l'insert.

La synthèse du premier brin d'ADNc se fait par élongation de l'oligonucléotide FC106, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC106 et FC107 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 2076 pb.

Ce fragment est digéré par EcoRV puis par XbaI pour isoler, après électrophorèse en gel d'agarose, le fragment EcoRV-XbaI d'environ 2058 pb.

Ce fragment est ligaturé avec le plasmide d'expression pVR1012, préalablement digéré par XbaI et EcoRV, pour donner le plasmide pFC105 (6953 pb). Ce plasmide contient, sous le contrôle du promoteur précoce du cytomégalovirus humain ou hCMV-IE (human Cytomegalovirus Immediate Early), un insert codant pour la polyprotéine prM-M-E.

### Exemple 8 : Construction du plasmide pFC106

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP » ou « RT-PCR ») est réalisée avec 50 µl de la suspension d'ARN viral du virus de la fièvre du Nil NY99 (exemple 2) et avec les oligonucléotides suivants :
FC108 (36 mer) (SEQ ID NO:10)
5' TTTTTTGATATCATGTATAATGCTGATATGATTGAC 3'
et FC109 (36 mer) (SEQ ID NO:11)
5' TTTTTTTCTAGATTAACGTTTTCCCGAGGCGAAGTC 3'.

Ce couple d'oligonucléotides permet l'incorporation d'un site de restriction EcoRV et d'un site de restriction XbaI, d'un codon ATG initiateur en 5' et d'un codon stop en 3' de l'insert.

La synthèse du premier brin d'ADNc se fait par élongation de l'oligonucléotide FC109, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC108 et FC109 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 2973 pb. '

Ce fragment est digéré par EcoRV puis par XbaI pour isoler, après électrophorèse en gel d'agarose, le fragment EcoRV-XbaI d'environ 2955 pb.

Ce fragment est ligaturé avec le plasmide d'expression pVR1012, préalablement digéré par XbaI et EcoRV, pour donner le plasmide pFC106 (7850 pb). Ce plasmide contient, sous le contrôle du promoteur précoce du cytomégalovirus humain ou hCMV-IE (human Cytomegalovirus Immediate Early), un insert codant pour la polyprotéine NS2A-NS2B-NS3.

### Exemple 9 : construction du plasmide donneur pour l'insertion dans le site C5 du virus canarypox ALVAC

La figure 16 du brevet US-A-5,756,103 montre la séquence d'un fragment de 3199 pb de l'ADN génomique du virus canarypox. L'analyse de cette séquence a révélé un cadre ouvert de lecture (COL) qui a été appelé C5L, qui commence à la position 1538 et se termine à la position 1859. La construction d'un plasmide d'insertion aboutissant à la délétion du COL C5L et à son remplacement par un site de clonage multiple flanqué de signaux d'arrêt de transcription et de traduction a été réalisée comme décrit cri-après.

Une réaction ACP a été réalisée à partir de la matrice constituée par l'ADN génomique du virus canarypox et avec les oligonucléotides suivants :
C5A1 (42 mer) (SEQ ID NO:12):

pour isoler un fragment ACP de 223 pb (fragment B).

Une réaction ACP a été réalisée à partir de la matrice constituée par l'ADN génomique du virus canarypox et avec les Oligonucléotides suivants :
C5C1 (72 mer) (SEQ ID NO :14) :

et C5D1 (45 mer) (SEQ ID NO :15) :
5' GATGATGGTACCGTAAACAAATATAATGAAAAGTATTCTAAACTA 3'
pour isoler un fragment ACP de 482 pb (fragment C).

Les fragments B et C ont été hybridés ensemble pour servir de matrice à une réaction ACP réalisée avec les oligonucléotides C5A1 (SEQ ID NO :12) et C5D1 (SEQ ID NO :15) pour générer un fragment ACP de 681 pb. Ce fragment a été digéré par les enzymes de restriction SacI et Kpnl, pour isoler, après électrophorèse en gel d'agarose, un fragment Sacl-Kpnl de 664 pb. Ce fragment a été ligaturé avec le vecteur pBlueScript^{®} Il SK+ (Stratagene, La Jolla, CA, USA, Cat # 212205), préalablement digéré par les enzymes de restriction SacI et KpnI, pour donner le plasmide pC5L. La séquence de ce plasmide a été vérifiée par séquençage. Ce plasmide contient 166 pb de séquences situées en amont du COL C5L (« bras flanquant gauche C5 »), un signal vaccine d'arrêt précoce de transcription, des codons stops dans les 6 phases de lecture, un site de clonage multiple contenant les sites dé restriction SmaI, PstI, XhoI et EcoRI, et enfin 425 pb de séquences situées en aval du COL C5L (« bras flanquant droit C5 »).

Le plasmide pMP528HRH (Perkus M. et al. J. Virol. 1989. 63. 3829-3836) a été utilisé comme matrice pour amplifier la séquence complète du promoteur vaccine H6 (N° d'accès GenBank M28351) avec les oligonucléotides suivants :
JCA291 (34 mer) (SEQ ID NO :16)
5' AAACCCGGGTTCTTTATTCTATACTTAAAAAGTG 3'
et JCA292 (43 mer) (SEQ ID NO :17)
5' AAAAGAATTCGTCGACTACGATACAAACTTAACGGATATCGCG 3' pour amplifier un fragment ACP de 149 pb. Ce fragment a été digéré par les enzymes de restriction SmaI et EcoRI pour isoler, après électrophorèse en gel d'agarose, un fragment de restriction SmaI-EcoRI de 138 pb. Ce fragment a alors été ligaturé avec le plasmide pC5L, préalablement digéré par Smal et EcoRI, pour donner le plasmide pFC107.

### Exemple 10 : construction du virus recombiné vCP1712

Une réaction ACP a été réalisée en utilisant le plasmide pFC105 (exemple 7) comme matrice et les oligonucléotides suivants :
FC110 (33 mer) (SEQ ID NO :18):
5' TTTTCGCGAACCGGAATTGCAGTCATGATTGGC 3'
et FC111 (39 mer) (SEQ ID NO :19):
5' TTTTGTCGACGCGGCCGCTTAAGCGTGCACGTTCACGGA 3'
pour amplifier un fragment ACP d'environ 2079 pb. Ce fragment a été digéré par les enzymes de restriction NruI et SalI pour isoler, après électrophorèse en gel d'agarose, un fragment dé restriction NruI-SalI d'environ 2068 pb. Ce fragment a été ensuite ligaturé avec le plasmide pFC107 (exemple 9) préalablement digéré par les enzymes de restriction NruI et SalI pour donner le plasmide pFC108.

Le plasmide pFC108 a été linéarisé par Notl, puis transfecté dans des cellules primaires d'embryons de poulets infectées avec du virus canarypox (souche ALVAC) selon la technique de précipitation au phosphate de calcium précédemment décrite (Panicali et Paoletti Proc. Nat. Acad. Sci. 1982. 79. 4927-4931 ; Piccini et al. In Methods in Enzymology. 1987. 153. 545-563. Eds. Wu R. and Grossman L. Academic Press). Des plages positives ont été sélectionnées sur la base d'une hybridation avec une sonde radiomarquée spécifique de la séquence nucléotidique de la glycoprotéine d'enveloppe E. Ces plages ont subi 4 cycles successifs de sélection/purification de plages jusqu'à ce qu'une population pure ait été isolée. Une plage représentative de la recombinaison *in vitro* entre le plasmide donneur pFC108 et le génome du virus canarypox ALVAC a alors été amplifiée et le stock de virus recombiné obtenu a été désigné vCP1712.

### Exemple 11 : construction du virus recombiné vCP1713

Le plasmide pFC104 (exemple 6) est digéré par les enzymes de restriction SalI et PmlI pour isoler, après électrophorèse en gel d'agarose, un fragment de restriction PmlI-SalI d'environ 2213 pb. Ce fragment est ligaturé avec le plasmide pFC107 (exemple 9) préalablement digéré par les enzymes de restriction NruI et SalI pour donner le plasmide pFC109.

Le plasmide pFC109 a été linéarisé par NotI, puis transfecté dans des cellules primaires d'embryons de poulets infectées avec du virus canarypox (souche ALVAC) selon la technique de l'exemple 10. Une plage représentative de la recombinaison *in vitro* entre le plasmide donneur pFC109 et le génome du virus canarypox ALVAC a été sélectionnée sur la basé d'une hybridation avec une sonde radiomarquée spécifique de la séquence nucléotidique de la glycoprotéine d'enveloppe E et a été ensuite amplifiée. Le stock de virus recombiné obtenu a été désigné vCP1713.

### Exemple 12 : construction du virus recombiné vCP1714

Le plasmide pFC103 (exemple 5) est digéré par les enzymes de restriction SalI et PmlI pour isoler, après électrophorèse en gel d'agarose, un fragment de restriction PmlI-SalI d'environ 1712 pb. Ce fragment est ligaturé avec le plasmide pFC107 (exemple 9) préalablement digéré par les enzymes de restriction NruI et SalI pour donner le plasmide pFC110.

Le plasmide pFC110 a été linéarisé par Notl, puis transfecté dans des cellules primaires d'embryons de poulets infectées avec du virus canarypox (souche ALVAC) selon la technique de l'exemple 10. Une plage représentative de la recombinaison *in vitro* entre le plasmide donneur pFC110 et le génome du virus canarypox ALVAC a été sélectionnée sur la base d'une hybridation avec une sonde radiomarquée spécifique de la séquence nucléotidique de la glycoprotéine d'enveloppe E et a été ensuite amplifiée. Le stock de virus recombiné obtenu a été désigné vCP1714.

### Exemple 13 : construction du virus recombiné vCP1715

Le plasmide pFC102 (exemple 4) est digéré par les enzymes de restriction Sali et Pmll pour isoler, après électrophorèse en gel d'agarose, un fragment de restriction PmlI-SalI d'environ 434 pb. Ce fragment est ligaturé avec le plasmide pFC107 (exemple 9) préalablement digéré par les enzymes de restriction NruI et SalI pour donner le plasmide pFC111.

Le plasmide pFC111 a été linéarisé par NotI, puis transfecté dans des cellules primaires d'embryons de poulets infectées avec du virus canarypox (souche ALVAC) selon la technique de l'exemple 10. Une plage représentative de la recombinaison *in vitro* entre le plasmide donneur pFC111 et le génome du virus canarypox ALVAC a été sélectionnée sur la base d'une hybridation avec une sonde radiomarquée spécifique de la séquence nucléotidique de la glycoprotéine de membrane M et a été ensuite amplifiée. Le stock de virus recombiné obtenu a été désigné vCP1715.

### Exemple 14 : construction du virus recombiné vCP1716

Le plasmide pFC101 (exemple 3) est digéré par les enzymes de restriction SalI et Pmll pour isoler, après électrophorèse en gel d'agarose, un fragment de restriction PmlI-SalI d'environ 484 pb. Ce fragment est ligaturé avec le plasmide pFC107 (exemple 9) préalablement digéré par les enzymes de restriction NruI et SalI pour donner le plasmide pFC112.

Le plasmide pFC112 a été linéarisé par NotI; puis transfecté dans des cellules primaires d'embryons de poulets infectées avec du virus canarypox (souche ALVAC) selon la technique de l'exemple 10. Une plage représentative de la recombinaison *in vitro* entre le plasmide donneur pFC112 et le génome du virus canarypox ALVAC a été sélectionnée sur la base d'une hybridation avec une sonde radiomarquée spécifique de la séquence nucléotidique de la glycoprotéine de pré-membrane prM et a été ensuite amplifiée. Le stock de virus recombiné obtenu a été désigné vCP1716.

### Exemple 15 : construction du plasmide donneur pour l'insertion dans le site C6 du virus canarypox ALVAC

La figure 4 du brevet WO-A-01/05934 montre la séquence d'un fragment de 3700 pb de l'ADN génomique du virus canarypox. L'analyse de cette séquence a révélé un cadre ouvert de lecture (COL) qui a été appelé C6L, qui commence à la position 377 et se termine à la position 2254. La construction d'un plasmide d'insertion aboutissant à la délétion du COL C6L et à son remplacement par un site de clonage multiple flanqué de signaux d'arrêt de transcription et de traduction a été réalisée comme décrit ci-après.

Une réaction ACP a été réalisée à partir de la matrice constituée par l'ADN génomique du virus canarypox et avec les oligonucléotides suivants :
C6A1 (42 mer) (SEQ ID NO :20) :
5' ATCATCGAGCTCGCGGCCGCCTATCAAAAGTCTTAATGAGTT 3'
et C6B1 (73 mer) (SEQ ID NO :21) :
5' GAATTCCTCGAGCTGCAGCCCGGGTTTTTATAGCTAATTAGTCAT TTTTCGTAAGTAAGTATTTTTATTTAA 3'
pour isoler un fragment ACP de 432 pb (fragment D).

Une réaction ACP a été réalisée à partir de la matrice constituée par l'ADN génomique du virus canarypox et avec les oligonucléotides suivants :
C6C1 (72 mer) (SEQ ID NO :22) :
5'CCCGGGCTGCAGCTCGAGGAATTCTTTTTATTGATTAACTAGTCA AATGAGTATATATAATTGAAAAAGTAA 3'
et C6D1 (45 mer) (SEQ ID NO :23) :
5' GATGATGGTACCTTCATAAATACAAGTTTGATTAAACTTAAGTTG 3'
pour isoler un fragment ACP de 1210 pb (fragment E).

Les fragments D et E ont été hybridés ensemble pour servir de matrice à une réaction ACP réalisée avec les oligonucléotides C6A1 (SEQ ID NO :20) et C6D1 (SEQ ID NO :23) pour générer un fragment ACP de 1630 pb. Ce fragment a été digéré par les enzymes de restriction SacI et KpnI, pour isoler, après électrophorèse en gel d'agarose, un fragment SacI-KpnI de 1613 pb. Ce fragment a été ligaturé avec le vecteur pBlueScript® II SK+ (Stratagene_{,} La Jolla, CA, USA, Cat # 212205), préalablement digéré par les enzymes de restriction SacI et KpnI, pour donner le plasmide pC6L. La séquence de ce plasmide a été vérifiée par séquençage. Ce plasmide contient 370 pb de séquences situées en amont du COL C6L (« bras flanquant gauche C6 »), un signal vaccine d'arrêt précoce de transcription, des codons stops dans les 6 phases de lecture, un site de clonage multiple contenant les sites de restriction Smal, PstI, XhoI et EcoRI, et enfin 1156 pb de séquences situées en aval du COL C6L (« bras flanquant droit C6 »).

Le plasmide pMPIVC (Schmitt J. F. C. et al., J. Virol., 1988, 62, 1889-1897; Saiki R. K. et al., Science, 1988, 239, 487-491) a été utilisé comme matrice pour amplifier la séquence complète du promoteur vaccine I3L avec les oligonucléotides suivants :
FC112 (33 mer) (SEQ ID NO :24) :
5' AAACCCGGGCGGTGGTTTGCGATTCCGAAATCT 3'
et FC113 (43 mer) (SEQ ID NO :25) :
5' AAAAGAATTCGGATCCGATTAAACCTAAATAATTGTACTTTGT 3'
pour amplifier un fragment ACP de 151 pb. Ce fragment a été digéré par les enzymes de restriction SmaI et EcoRI pour isoler, après électrophorèse en gel d'agarose, un fragment de restriction SmaI-EcoRI d'environ 136 pb. Ce fragment a alors été ligaturé avec le plasmide pC6L, préalablement digéré par SmaI et EcoRI, pour donner le plasmide pFC113.

### Exemple 16 : construction des virus recombinés vCP1717 et vCP1718

Une réaction ACP a été réalisée en utilisant le plasmide pFC106 (exemple 8) comme matrice et les oligonucléotides suivants :
FC114 (33 mer) (SEQ ID NO :26) :
5'TTTCACGTGATGTATAATGCTGATATGATTGAC 3'
et FC115 (42 mer) (SEQ ID NO :27) :
5'TTTTGGATCCGCGGCCGCTTAACGTTTTCCCGAGGCGAAGTC 3'
pour amplifier un fragment ACP d'environ 2973 pb. Ce fragment a été digéré avec les enzymes de restriction PmlI et BamHI pour isoler, après électrophorèse en gel d'agarose, le fragment de restriction Pmll-BamHl d'environ 2958 pb (fragment F). Le plasmide pFC113 (exemple 15) a été digéré par les enzymes de restriction PmlI et BamHI pour isoler, après électrophorèse en gel d'agarose, le fragment de restriction PmlI-BamHI d'environ 4500 pb (fragment G). Les fragments F et G ont alors été ligaturés ensemble pour donner le plasmide pFC114.

Le plasmide pFC114 a été linéarisé par NotI, puis transfecté dans des cellules primaires d'embryons de poulets infectées avec du virus canarypox vCP1713 (exemple 11) selon la technique de précipitation au phosphate de calcium précédemment décrite (Panicali et Paoletti Proc. Nat. Acad. Sci. 1982. 79. 4927-4931 ; Piccini et al. In Methods in Enzymology. 1987. 153. 545-563. Eds. Wu R. and Grossman L. Academic Press). Des plages positives ont été sélectionnées sur la base d'une hybridation avec une sonde radiomarquée spécifique de la séquence nucléotidique de la glycoprotéine d'enveloppe E. Ces plages ont subi 4 cycles successifs de sélection/purification de plages jusqu'à ce qu'une population pure ait été isolée. Une plage représentative de la recombinaison *in vitro* entre le plasmide donneur pFC114 et le génome du virus canarypox ALVAC a alors été amplifiée et le stock de virus recombiné obtenu a été désigné vCP1717.

Le plasmide pFC114 linéarisé par NotI a également servi à transfecter des cellules primaires d'embryons de poulets infectées avec du virus canarypox vCP1712 (exemple 10) selon la technique décrite ci-dessus. Le stock de virus recombiné ainsi obtenu a été désigné vCP1718.

### Exemple 17 : Construction du plasmide pFC115

L'ADN complémentaire (ADNc) du virus de la fièvre du Nil NY99 est synthétisé avec le kit « Gene Amp RNA PCR Kit » (Cat # N 808 0017, Perkin-Elmer, Norwalk, CT 06859, USA) en utilisant les conditions données par le fournisseur.

Une réaction de transcription inverse, suivie d'une réaction d'amplification en chaîne (Réaction « TI-ACP» ou « RT-PCR ») est réalisée avec 50 µl de la suspension d'ARN viral du virus de la fièvre du Nil NY99 (exemple 2) et avec les oligonucléotides suivants :
FC116 (39 mer) (SEQ ID NO :28)
5' TTTTTTGATATCATGACCGGAATTGCAGTCATGATTGGC 3'
et FC106 (33 mer) (SEQ ID NO :8).

Ce couple d'oligonucléotides permet l'incorporation d'un site de restriction EcoRV et d'un site de restriction XbaI, d'un codon initiateur en 5' et d'un codon stop en 3' de l'insert

La synthèse du premier brin d'ADNc se fait par élongation de l'oligonucléotide FC106, après hybridation de ce dernier à la matrice d'ARN.

Les conditions de synthèse du premier brin d'ADNc sont une température de 42°C pendant 15 min, puis de 99°C pendant 5 min, et enfin de 4°C pendant 5 min. Les conditions de la réaction ACP en présence du couple d'oligonucléotides FC106 et FC116 sont une température de 95°C pendant 2 min, puis 35 cycles (95°C pendant 1 min, puis 62°C pendant 1 min, et 72°C pendant 2 min), et enfin 72°C pendant 7 min pour produire un fragment de 2079 pb.

Ce fragment est digéré par EcoRV puis par Xbal pour isoler, après électrophorèse en gel d'agarose, le fragment EcoRV-Xbal d'environ 2061 pb.

Ce fragment est ligaturé avec le plasmide d'expression pVR1012, préalablement digéré par Xbal et EcoRV, pour donner le plasmide pFC115 (6956 pb). Ce plasmide contient, sous le contrôle du promoteur précoce du cytomégalovirus humain ou hCMV-IE (human Cytomegalovirus Immédiate Early), un insert codant pour la polyprotéine prM-M-E.

### Exemple 18 : construction des virus recombinés vCP2017

Une réaction ACP a été réalisée en utilisant le plasmide pFC115 (exemple 17) comme matrice et les oligonucléotides suivants :
FC117 (36 mer) (SEQ ID NO :29) :
5' TTTTCGCGAATGACCGGAATTGCAGTCATGATTGGC 3'
et FC111 (39 mer) (SEQ ID NO :19)
pour amplifier un fragment ACP d'environ 2082 pb. Ce fragment a été digéré par les enzymes de restriction NruI et SalI pour isoler, après électrophorèse en gel d'agarose, un fragment de restriction NruI-SalI d'environ 2071 pb. Ce fragment a été ensuite ligaturé avec le plasmide pFC107 (exemple 9) préalablement digéré par les enzymes de restriction Nrul et SalI pour donner le plasmide pFC116.

Le plasmide pFC116 a été linéarisé par NotI, puis transfecté dans des cellules primaires d'embryons de poulets infectées avec du virus canarypox (souche ALVAC) selon la technique de l'exemple 10. Une plages représentative de la recombinaison *in vitro* entre le plasmide donneur pFC116 et le génome du virus canarypox ALVAC a été sélectionnée sur la base d'une hybridation avec une sonde radiomarquée spécifique de la séquence nucléotidique de la glycoprotéine d'enveloppe E et a été ensuite amplifiée. Le stock de virus recombiné obtenu a été désigné vCP2017.

### Exemple 19 : Production de vaccins recombinés

Pour la préparation de vaccins équins, le virus recombiné canarypox vCP1712 (exemple 10) est adjuvé avec des solutions de carbomère, à savoir Carbopol™ 974P fabriqué par BF Goodrich, Ohio, USA (poids moléculaire d'environ 3 000 000).

Une solution stock à 1,5 % de Carbopol™ 974P est initialement préparée dans de l'eau distillée contenant 1 g/l de chlorure de sodium. Cette solution stock est alors utilisée pour l'élaboration d'une solution à 4 mg/ml de Carbopol™ 974P en eau physiologique. La solution stock est mélangée au volume adéquat d'eau physiologique, soit en une étape unique soit en plusieurs étapes successives, la valeur du pH est ajustée à chaque étape avec une solution d'hydroxyde de sodium 1N (ou encore plus concentrée) afin d'obtenir une valeur finale de pH de 7,3-7,4.

La solution de Carbopol™ 974P prête à l'emploi ainsi obtenue est utilisée pour reprendre des virus recombinés lyophilisés ou pour diluer des solutions stocks concentrées de virus recombinés. Par exemple, pour obtenir une suspension virale contenant 10⁸ pfu par dose de 1 ml, on dilue une solution virale stock de manière à obtenir un titre de 10^{8,3} pfu/ml, puis on dilue à parties égales avec ladite solution de Carbopol™ 974P prête à l'emploi à 4 mg/ml.

Des vaccins recombinés peuvent également être produits avec des virus recombinés canarypox vCP1713 (exemple 11) ou vCP1717 (exemple 16) ou vCP1718 (exemple 16) ou vCP2017 (exemple 18) ou un mélange des trois virus canarypox vCP1714 (exemple 12), vCP1715 (exemple 13) et vCP1716 (exemple 14) selon la technique décrite ci-dessus.

### Exemple 20 : Production de vaccins ADN pour les équins

Une solution d'ADN contenant le plasmide pFC104 (exemple 6) est concentrée par précipitation éthanolique comme décrit dans Sambrook et al (1989). Le culot d'ADN est repris par une solution de NaCl 0.9% de façon à obtenir une concentration de 1 mg/ml. Une solution de DMRIE-DOPE à 0,75 mM est préparée par reprise d'un lyophilisat de DMRIE-DOPE par un volume adapté d'H₂O stérile.

La formation des complexes ADN plasmidique-lipide est réalisée par dilution à parties égales de la solution à 0.75 mM de DMRIE-DOPE (1:1) par la solution d'ADN à 1 mg/ml dans NaCl 0.9%. La solution d'ADN est introduite progressivement à l'aide d'une aiguille sertie 26G le long de la paroi du flacon contenant la solution de lipide cationique de façon à éviter la formation de mousse. On procède à une agitation douce dès que les deux solutions sont mélangées. On obtient en final une composition comprenant 0,375 mM de DMRIE-DOPE et 500 µg/ml de plasmide.

Il est souhaitable que l'ensemble des solutions utilisées soient à température ambiante pour l'ensemble des opérations décrites ci-dessus. On laisse la complexation ADN/DMRIE-DOPE se mettre en place à température ambiante pendant 30 minute avant de procéder à l'immunisation des animaux.

Des vaccins ADN peuvent également être produits avec des solutions d'ADN contenant les plasmides pFC104 (exemple 6) et pFC106 (exemple 8), ou contenant les plasmides pFC105 (exemple 7) et pFC106, les plasmides pFC115 (exemple 17) et pFC106, ou contenant les plasmides pFC101, pFC102 et pFC103 (exemples 3 à 5), ou contenant le plasmide pFC105 ou pFC115 selon la technique décrite dans le présent exemple.

### Exemple 21 : Tests d'expression in vitro

L'expression des protéines WN est testée pour chaque construction par les méthodes classiques d'immunofluorescence indirecte et de Western Blot.

Ces tests sont effectués sur des plaques 96 puits contenant les cellules CHO cultivées en monocouches et transfectées par des plasmides ou contenant les cellules CEF cultivées en monocouches et infectées par des virus recombinés.

Les protéines WN sont détectées par utilisation de sérums de chevaux ou de poulets infectés et d'anti-sérums marqués.

La taille des fragments obtenus après migration sur gel d'agarose est comparée à celles attendues.

### Exemple 22 : Efficacité sur animaux

Les vaccins recombinés et les vaccins plasmidiques sont injectés à deux reprises à environ deux semaines d'intervalle à des poulets EOPS, âgés d'environ 7 jours, non vaccinés, par voie intramusculaire sous un volume d'environ 0,1 ml. Un groupe témoin non vacciné est incorporé à l'étude.

Les poulets sont éprouvés par administration sous-cutanée dans le cou de 10³⁻⁴TCID₅₀ de virus WN pathogène.

Sont observés d'une part la virémie, la réponse en anticorps ainsi que la mortalité. Des autopsies sont pratiquées pour observer les lésions.

### Exemple 23 : Titrage des anticorps neutralisants anti-WNV.

Des séries de dilution sont réalisées pour chaque sérum avec une raison de 3 dans du milieu DMEM additionné de 10% de sérum de veau foetal dans des plaques à 96 puits type culture cellulaire. A 0,05 ml du sérum dilué est ajouté 0,05 ml de milieu de culture contenant approximativement 100 DICC₅₀/ml de WNV. Ce mélange est incubé pendant 2 heures à 37°C en étuve en atmosphère contenant 5% CO₂.

0,15 ml d'une suspension de cellules VERO contenant environ 100 000 cellules par ml est ensuite ajouté à chaque mélange. L'effet cytopathique (ECP) est observé par microscopie à contraste de phase après 4-5 jours de culture à 37°C en atmosphère contenant 5% CO₂. Les titres neutralisants de chaque sérum sont calculés d'après la méthode de Kärber. Les titres sont donnés sous la forme de la dilution la plus importante inhibant l'effet cytopathique pour 50 % des puits. Les titres sont exprimés en log10 VN50. Chaque sérum est titré au moins deux fois, de préférence quatre fois.

### Exemple 24 : Essai sur chevaux de vCP2017.

Des vaccins recombinés contenant ACP2017 (exemple 18) formulés extemporanément avec 1 ml de l'adjuvant carbopol® 974P (4 mg/ml) ont été injectés à deux reprises à 35 jours d'intervalle à des chevaux, âgés de plus de 3 mois, non vaccinés, par voie intramusculaire sous un volume d'environ 1 ml. Trois groupes d'animaux ont été vaccinés, avec des doses de 10^{5,8} DICC₅₀ (soit 10^{5,64} pfu) pour le groupe 1, 10^{6,8} DICC₅₀ (soit 10^{6,64} pfu) pour le groupe 2 et 10^{7,8} DICC₅₀ (soit 10^{7,64} pfu) pour le groupe 3. Un groupe témoin non vacciné a été incorporé à l'étude.

La sérologie a été observée. Les titres en anticorps neutralisants ont été établis et exprimés en log10 VN50 comme indiqué à l'exemple 23.

| **Groupe** | **Titres à J0** | **Titres à J35** | **Titres à J49** |
|---|---|---|---|
| 1 | <0,6 | <0,78 | 2,66 |
| 2 | <0,6 | 1,14 | 2,58 |
| 3 | <0,6 | 1,16 | 2,26 |
| témoin | <0,6 | <0,6 | <0,6 |

## Revendications

1. Composition immunogène pour une utilisation dans la protection des équins contre le virus de la fièvre du nile (WN) ou vaccin pour une utilisation dans la protection des équins contre le virus de la fièvre du Nile (WN), comprenant un ou plusieurs virus recombinés avipox et exprimant les protéines prM, M et E du virus WN, les virus recombinés comprenant un ou plusieurs polynucléotides codant pour une ou plusieurs protéines prM, M et E, et un véhicule ou excipient pharmaceutiquement acceptable.

2. Composition immunogène ou vaccin pour une utilisation selon la revendication 1, **caractérisé en ce qu'**il comprend un virus recombiné avipox qui exprime les trois protéines prM, M et E.

3. Composition immunogène ou vaccin pour une utilisation selon la revendication 1, **caractérisé en ce qu'**il comporte un virus recombiné avipox qui comprend un polynucléotide formant une phase de lecture codant pour prM-M-E.

4. Composition immunogène ou vaccin selon la revendication 1, 2 ou 3 pour une utilisation selon la revendication 1 , **caractérisé en ce que** le virus recombiné est un canarypox.

5. Composition immunogène ou vaccin selon la revendication 1, 2 ou 3 pour une utilisation selon la revendication 1, **caractérisé en ce que** 5. Composition immunogène ou vaccin selon la revendication 1, 2 ou 3 pour une utilisation selon la revendication 1, **caractérisé en ce que** le virus recombiné est un fowlpox.

6. Composition immunogène ou vaccin selon l'une des revendications 1 à 5 pour une utilisation selon la revendication 1, **caractérise en ce que** le polynucléotide code aussi pour un peptide signal.

7. Composition immunogène ou vaccin selon la revendication 6 pour la une utilisation selon la revendication, **caractérisé en ce qu'**il s'agit d'un peptide signal du virus WN.

8. Composition immunogène ou vaccin selon la revendication 6 pour une utilisation selon la revendication 1, **caractérisé en ce qu'**il s'agit d'un peptide signal hétérologue.

9. Composition immunogène ou vaccin selon la revendication 8 pour une utilisation selon la revendication 1, **caractérisé en ce qu'**il s'agit du peptide signal de l'activateur tissulaire du plasminogène humain.

10. Composition immunogène ou vaccin selon l'une quelconque des revendications 1 à 9 pour une utilisation selon la revendication 1, **caractérisé en ce qu'**il comprend un autre polynucléotide codant pour une protéine d'une autre souche de virus WN et/ou d'un autre agent pathogène et/ou pour une cytokine.

11. Composition immunogène ou vaccin selon l'une quelconque des revendications 1 à 10 pour une utilisation selon la revendication 1, **caractérisé en ce qu'**il comprend un autre vecteur d'expression comprenant un autre polynucléotide codant pour une protéine d'une autre souche de virus WN et/ou d'un autre agent pathogène et/ou pour une cytokine.

12. Composition immunogène ou vaccin selon l'une quelconque des revendications 1 à 11 pour une utilisation selon la revendication 1, **caractérisé en ce qu'**il comprend en outre un adjuvant.

13. Composition immunogène ou vaccin selon la revendication 12 pour une utilisation selon la revendication 1, **caractérisé en ce que** l'adjuvant est choisi parmi (1) les polymères de l'acide acrylique ou méthacrylique et les polymères d'anhydride maléique et de dérivé alcényle, (2) les séquences immunostimulatrices, (3) une émulsion huile-dans-l'eau, (4) les lipides cationiques contenant un sel d'ammonium quaternaire, (5) les cytokines.

14. Composition immunogène ou vaccin selon la revendication 13 pour une utilisation selon la revendication 1, **caractérisé en ce que** l'adjuvant est un carbomère.

15. Composition immunogène ou vaccin selon la revendication 10 ou 13 pour une utilisation selon la revendication 1, **caractérisé en ce que** la cytokine est une cytokine équine ou une cytokine aviaire.

16. Composition immunogène ou vaccin selon la revendication 15 pour une utilisation selon la revendication 1, **caractérisé en ce que** la cytokine est choisie parmi interleukine 18, interleukine 12, interleukine 15, MIP-1a, GM-CSF.

17. Composition immunogène ou vaccin selon la revendication 15 pour une utilisation selon la revendication 1, **caractérisé en ce que** la cytokine est le GM-CSF équin.

18. Composition immunogène ou vaccin selon l'une quelconque des revendications 1 à 17 pour une utilisation selon la revendication 1, formant une composition immunogène ou un vaccin multivalent, **caractérisé en ce qu'**il comprend un composant de composition immunogène ou vaccin contre un autre agent pathogène.

19. Composition immunogène ou vaccin selon la revendication 18 pour une utilisation selon la revendication 1, **caractérisé en ce que** le composant contre un autre agent pathogène comporte de l'antigène sous forme de sous-unité, sous-forme d'agent inactivé ou sous-forme d'agent atténué.

20. Composition immunogène ou vaccin selon la revendication 18 ou 19 pour une utilisation selon la revendication 1, **caractérisé en ce qu'**il comprend un composant de composition immunogène ou vaccin contre le virus de la rhinopneumonie équine, contre le virus de la grippe équine, contre le virus de l'encéphalite de l'Est, contre le virus de l'Encéphalite de l'Ouest, contre le virus de l'Encéphalite Vénézuélienne, contre Clostridium tetani, et leurs mélanges.

## Patentansprüche

1. Immunogene Zusammensetzung zur Verwendung beim Schutz von Pferden vor dem West-Nil-Virus (WNV) oder Impfstoff zur Verwendung beim Schutz von Pferden vor dem West-Nil-Virus (WNV), die/der einen oder mehrere rekombinante Vogelpockenviren, die Proteine prM, M und E des WN-Virus exprimieren, und einen pharmazeutisch verträglichen Träger umfasst, wobei die rekombinanten Viren ein oder mehrere Polynucleotide umfassen, die ein oder mehrere prM-, M- und E-Proteine codieren.

2. Immunogene Zusammensetzung oder Impfstoff zur Verwendung nach Anspruch 1, **dadurch gekennzeichnet, dass** sie/er ein rekombinantes Vogelpockenvirus umfasst, das die drei Proteine prM, M und E exprimiert.

3. Immunogene Zusammensetzung oder Impfstoff zur Verwendung nach Anspruch 1, **dadurch gekennzeichnet, dass** sie/er ein rekombinantes Vogelpockenvirus enthält, das ein Polynucleotid umfasst, das einen Leserahmen bildet, der prM-M-E codiert.

4. Immunogene Zusammensetzung oder Impfstoff nach Anspruch 1, 2 oder 3 zur Verwendung nach Anspruch 1, **dadurch gekennzeichnet, dass** das rekombinante Virus ein Kanarienpockenvirus ist.

5. Immunogene Zusammensetzung oder Impfstoff nach Anspruch 1, 2 oder 3 zur Verwendung nach Anspruch 1, **dadurch gekennzeichnet, dass** das rekombinante Virus ein Geflügelpockenvirus ist.

6. Immunogene Zusammensetzung oder Impfstoff nach einem der Ansprüche 1 bis 5 zur Verwendung nach Anspruch 1, **dadurch gekennzeichnet, dass** das Polynucleotid auch ein Signalpeptid codiert.

7. Immunogene Zusammensetzung oder Impfstoff nach Anspruch 6 zur Verwendung nach Anspruch 1, **dadurch gekennzeichnet, dass** es sich um ein Signalpeptid des West-Nil-Virus handelt.

8. Immunogene Zusammensetzung oder Impfstoff nach Anspruch 6 zur Verwendung nach Anspruch 1, **dadurch gekennzeichnet, dass** es sich um ein heterologes Signalpeptid handelt.

9. Immunogene Zusammensetzung oder Impfstoff nach Anspruch 8 zur Verwendung nach Anspruch 1, **dadurch gekennzeichnet, dass** es sich um das Signalpeptid des humanen gewebespezifischen Plasminogen-Aktivators handelt.

10. Immunogene Zusammensetzung oder Impfstoff nach einem der Ansprüche 1 bis 9 zur Verwendung nach Anspruch 1, **dadurch gekennzeichnet, dass** sie/er ein weiteres Polynucleotid umfasst, das ein Protein eines anderen West-Nil-Virusstamms und/oder eines anderen Pathogens und/oder ein Cytokin codiert.

11. Immunogene Zusammensetzung oder Impfstoff nach einem der Ansprüche 1 bis 10 zur Verwendung nach Anspruch 1, **dadurch gekennzeichnet, dass** sie/er einen weiteren Expressionsvektor umfasst, der ein weiteres Polynucleotid umfasst, das ein Protein eines anderen West-Nil-Virusstamms und/oder eines anderen Pathogens und/oder ein Cytokin codiert.

12. Immunogene Zusammensetzung oder Impfstoff nach einem der Ansprüche 1 bis 11 zur Verwendung nach Anspruch 1, **dadurch gekennzeichnet, dass** sie/er des Weiteren ein Adjuvans umfasst.

13. Immunogene Zusammensetzung oder Impfstoff nach Anspruch 12 zur Verwendung nach Anspruch 1, **dadurch gekennzeichnet, dass** das Adjuvans ausgewählt ist aus (1) Polymeren der Acrylsäure oder Methacrylsäure und Maleinsäureanhydridpolymeren und Alkenylderivatpolymeren, (2) immunstimulatorischen Sequenzen, (3) einer ÖI-in-Wasser-Emulsion, (4) ein quarternäres Ammoniumsalz enthaltenden kationischen Lipiden, (5) Cytokinen.

14. Immunogene Zusammensetzung oder Impfstoff nach Anspruch 13 zur Verwendung nach Anspruch 1, **dadurch gekennzeichnet, dass** das Adjuvans ein Carbomer ist.

15. Immunogene Zusammensetzung oder Impfstoff nach Anspruch 10 oder 13 zur Verwendung nach Anspruch 1, **dadurch gekennzeichnet, dass** das Cytokin ein Pferdecytokin oder ein Vogelcytokin ist.

16. Immunogene Zusammensetzung oder Impfstoff nach Anspruch 15 zur Verwendung nach Anspruch 1, **dadurch gekennzeichnet, dass** das Cytokin ausgewählt ist aus Interleukin 18, Interleukin 12, Interleukin 15, MIP-1a, GM-CSF.

17. Immunogene Zusammensetzung oder Impfstoff nach Anspruch 15 zur Verwendung nach Anspruch 1, **dadurch gekennzeichnet, dass** das Cytokin GM-CSF des Pferdes ist.

18. Immunogene Zusammensetzung oder Impfstoff nach einem der Ansprüche 1 bis 17 zur Verwendung nach Anspruch 1, das eine multivalente immunogene Zusammensetzung oder einen multivalenten Impfstoff bildet, **dadurch gekennzeichnet, dass** sie/er eine Komponente einer immunogenen Zusammensetzung oder eines Impfstoffs gegen ein weiteres Pathogen umfasst.

19. Immunogene Zusammensetzung oder Impfstoff nach Anspruch 18 zur Verwendung nach Anspruch 1, **dadurch gekennzeichnet, dass** die Komponente gegen ein weiteres Pathogen das Antigen in Form einer Untereinheit, einer inaktivierten Untereinheit des Agens oder einer attenuierten Untereinheit des Agens umfasst.

20. Immunogene Zusammensetzung oder Impfstoff nach Anspruch 18 oder 19 zur Verwendung nach Anspruch 1, **dadurch gekennzeichnet, dass** sie/er eine Komponente einer immunogenen Zusammensetzung oder eines Impfstoffs gegen das Virus der Rhinopneumonie des Pferdes, gegen das Pferdeinfluenza-Virus, gegen das Östliche-Pferdeenzephalitis-Virus, gegen das Westliche-Pferdeenzephalitis-Virus, gegen das Venezolanische-Pferdeenzephalitis-Virus, gegen *Clostridium tetani* und Mischformen davon umfasst.

## Claims

1. An immunogenic composition for use in protecting equines against West Nile (WN) virus or vaccine for use in protecting equines against West Nile (WN) virus, comprising one or several recombinant avipox viruses, and expressing the prM, M and E proteins of the WN virus, said recombinant viruses comprising one or several polynucleotides coding for one or several of the prM, M and E proteins, and a pharmaceutically acceptable vehicle or excipient.

2. Immunogenic composition or vaccine for use according to claim 1, **characterized in that** it comprises a recombinant avipox virus expressing the three proteins prM, M and E.

3. Immunogenic composition or vaccine for use according to claim 1, **characterized in that** it comprises a recombinant avipox virus comprising a polynucleotide forming a reading frame for prM-M-E.

4. Immunogenic composition or vaccine according to claim 1, 2 or 3 for use according to claim 1, **characterized in that** the recombinant virus is a canarypox.

5. Immunogenic composition or vaccine according to claim 1, 2 or 3 for use according to claim 1, **characterized in that** the recombinant virus is a fowlpox.

6. Immunogenic composition or vaccine according to any one of claims 1 to 5 for use according to claim 1, **characterized in that** the polynucleotide also codes for a signal peptide.

7. Immunogenic composition or vaccine according to claim 6 for use according to claim 1, **characterized in that** the signal peptide is a signal peptide from the WN virus.

8. Immunogenic composition or vaccine according to claim 6 for use according to claim 1, **characterized in that** the signal peptide is a heterologous signal peptide.

9. Immunogenic composition or vaccine according to claim 8 for use according to claim 1, **characterized in that** the signal peptide is the signal peptide from human tissue plasminogen activator.

10. Immunogenic composition or vaccine according to any one of claims 1 to 9 for use according to claim 1, **characterized in that** it comprises another polynucleotide coding for a protein from another strain of WN virus and/or another pathogenic agent and/or for a cytokine.

11. Immunogenic composition or vaccine according to any one of claims 1 to 10 for use according to claim 1, **characterized in that** it comprises another expression vector comprising another polynucleotide coding for a protein from another strain of WN virus and/or another pathogenic agent and/or for a cytokine.

12. Immunogenic composition or vaccine according to any one of claims 1 to 11 for use according to claim 1, **characterized in that** it further comprises an adjuvant.

13. Immunogenic composition or vaccine according to claim 12 for use according to claim 1, **characterized in that** the adjuvant is selected among (1) polymers of acrylic or methacrylic acid, maleic anhydride and alkenyl derivative polymers, (2) immunostimulating sequences, (3) an oil in water emulsion, (4) cationic lipids containing a quaternary ammonium salt, (5) cytokines.

14. Immunogenic composition or vaccine according to claim 13 for use according to claim 1, **characterized in that** the adjuvant is a carbomer.

15. Immunogenic composition or vaccine according to claim 10 or 13 for use according to claim 1, **characterized in that** the cytokine is an equine cytokine or an avian cytokine.

16. Immunogenic composition or vaccine according to claim 15 for use according to claim 1, **characterized in that** the cytokine is selected among interleukin 18, interleukin 12, interleukin 15, MIP-1α, GM-CSF.

17. Immunogenic composition or vaccine according to claim 15 for use according to claim 1, **characterized in that** the cytokine is equine GM-CSF.

18. Immunogenic composition or vaccine according to any one of claims 1 to 17 for use according to claim 1, forming a multivalent immunogenic composition or vaccine, **characterized in that** it comprises an immunogenic composition or vaccine component against another pathogenic agent.

19. Immunogenic composition or vaccine according to claim 18 for use according to claim 1, **characterized in that** the immunogenic composition or vaccine component against another pathogenic agent comprises the antigen in the form of subunits, in the form of an inactivated agent, or in the form of an attenuated agent.

20. Immunogenic composition or vaccine according to claim 18 or 19 for use according to claim 1, **characterized in that** it comprises a immunogenic composition or vaccine component against equine rhinopneumonia virus, against equine influenza virus, against East encephalitis virus, against West encephalitis virus, against Venezuela encephalitis virus, against *Clostridium tetani,* and mixtures thereof.
